# EUROPEAN PATENT APPLICATION

(11) **EP 0 824 910 A2**
(43) Date of publication of application: **25.02.1998**
(21) Application number: 97202849.2
(22) Date of filing: 21.05.1993
(51) Int. Cl.: A61K 7/28, C12N 9/64, C12N 9/48

(54) **Use of a Krill Enzyme for the Treatment of Dental Plaque**

(30) Priority: 22.05.1992 SE 9201628
(62) Divisional of application: 93910549.0
(71) Applicant: PHAIRSON MEDICAL, INC., Wilmington, Delaware (US)
(72) Inventor: Lindbloom, Ragnvald, 740 10 Almunge (SE); De Faire, Johan, 740 33 Vattholma (SE)
(74) Representative: Lunt, Mark George Francis

(57) **Abstract**

Non-immunogenic enzyme composition which has been isolated from antartic krill and exhibits both endo- and exo-peptidase activity, comprises essentially a single proteolytic enzyme having a molecular weight from 26,000 to 32,000, as determined by SDS-PAGE. The composition is useful in the manufacture of compositions for the treatment of dental plaque.

## Description

### Technical field

The present invention relates to new pharmaceutical uses of non-immunogenic proteinaceous substances and compositions which have enzymatic activity and which in a great number of clinical tests surprisingly have proven to provide a cure for an amazingly great variety of diseases. According to the the invention it is believed that these substances function in a phagocytosis-like manner, i.e. they seem to be able to distinguish between various particles and soluble matter regarded to be "normal" or "abnormal", respectively, in a particular environment or, differently expressed, seem to be able of recognizing, targeting and destroying divergent cells. It should, however, be emphasized that the invention is not intended to be restricted by any hypothetic mode of action expressed in the present specification.

### Description of Prior Art

One traditional way of looking upon proteolytic enzymes for medical use is to use them for cleansing of non-healing wounds covered with proteinaceous structures - as an alternative to surgical debridement, mainly in elderly patients with ulcera caused by circulation insufficiencies. See e.g. US-A-4,801,451 and US-A-4,963,491, which disclose a mixture of exo- and endopeptidase enzymes which have been isolated from antarctic krill (Euphasia superba) and its use for cleaning purposes, both pharmaceutical cleaning (US-A-4,801,451) and non-pharmaceutical cleaning (US-A-4,963,491). The only disclosed pharmaceutical cleaning is enzymatic debridement, viz. enzymatic cleansing of necrotic wounds. Disclosed uses for non-pharmaceutical purposes are i.a. as laundering agents, for renovation of old paintings etc. WO 85/04809 discloses the use of enzymes from antarctic krill as a digestion promotor. EP-A1-0170115 discloses the use of krill as a thrombus dissolvent.

Enzymes and enzyme mixtures derived from antarctic krill have been isolated and extensively studied as regards their biological and biochemical properties. Various procedures for isolating these enzymes have also been developed. See e.g. Anheller J-E., Hellgren L., Karistam B. and Vincent J. (1989): "Biochemical and biological profile of a new enzyme preparation from Antarctic krill (E. superba) suitable for debridement of ulcerative lesions", Arch. Dermatol. Res. 281, 105-110; Axelsen N.H., Kroll J. and Weeke B. (1973): "A manual of quantitative immunoelectrophoresis: methods and applications", Scan. J. Immunol. 2, Suppl.; Bucht A. and Karlstam B. (1986). "Immunological characterization of three highly purified trypsin-like enzymes from Antarctic krill (Euphausia superba), Biol. Chem. Hoppe-Seyler 367, 366 (Suppl.); Bucht A., Johansson, B. and Karlstam B. (1986): "Separation and identification of proteases from Antarctic krill (Euphausia superba)", 6th Int. Symp. HPLC Proteins, Peptides and Polynucleotides, Baden-Baden, pp. 34; Chen C.S., Yan T.R. and Chen H.Y. (1978): "Purification and properties of trypsin-like enzymes and a carboxypeptidase A from Euphausia superba", J. Food Biochem. 2, 349-366; Hellgren L., Karistam B., Mohr V. and Vincent J. (1991): "Krill enzymes - a new concept for efficient debridement of necrotic ulcers", Int. J. Dermatol. 30, 102-103; Karestam B.: "Crossed immunoelectrophoretic krill analysis of proteins from Antarctic krill (Euphausia superba) with special reference to serine proteinases" (submitted for publication); Kimoto K.K., Kusama S. and Murakami K. (1983); "Purification and characterization of serine proteinases from Euphausia superba", Agric. Biol. Chem. 47, 529-534; Lowry O.H., Rosebrough N.J., Farr A.L. and Randal R.J. (1951): "Protein measurement with the Folin phenol reagent", J. Biol. Chem. 193, 265-275; Moore S. and Stein W.H. (1963): "Chromatographic determination of amino acids by the use of automatic recording equipment", Methods Enzymol. 6, 819-831; Osnes K.K. and Mohr V. (1985): "On the purification and characterization of three anionic serine-type peptide hydrolases from Antarctic krill, Euphausia superba", Comp. Biochem. Physiol. 82B, 607-619; and Osnes K.K. and Mohr V. (1986): On the purification and characterization of exopeptidases from Antarctic krill, Euphausia superba", Comp. Biochem. Physiol. 83B, 445-458.

### Background of the invention

Our immune system is always on guard and active and its main functions are to protect and to preserve the integrity of our body by taking care of foreign invaders, old and worn-out cells, divergent cells and soluble matters that are regarded to be abnormal in the specific environment.

Phagocytes, granulocytes, macrophages and NK-cells, are our "old immunocells" and their mode of action for protection and preservation is phagocytosis, i.e. engulfing for example a germ inside themselves and releasing lysosomal enzymes that will kill and decompose the germ.

Lymphocytes, T-cells and B-cells are our "new immunoceils" and they present a variety of modes of action for protection and preservation. Some T-cells release toxins for killing of microbes, other deliver messages and control the intensity of response and B-cells produce antibodies. The old and new cells interact in a complex interplay for optimal protection of our body.

In case of a tissue damage the granulocytes appear at site within a few minutes and they immediately start to attack foreign invaders, damaged cells etc. Within a few hours macrophages, NK-cells, and T-cells have responded to the chemotactic signals from granulocytes at work and they enter into the area. A sophisticated interplay starts for scavenging of damaged tissue to final healing.

The sign of an activated immune defence can be seen as an inflammation which is caused by leaking toxins from different immunocells and microbes and the inflammation is healthy as long as it is kept at a low level. Sometimes the immune defence gets over-activated and an acute inflammation appears that is causing more harm than good, and if protracted the inflammation may become chronic which might be the starting point of an autoimmune condition.

The main reason for inflammations going astray is microbial injections and despite the manifold actions of our immune defence, microbes have developed means of selfprotection that in the long run could lead to very hazardous conditions.

The microbes may disguise themselves to irrecognition for the immunocells or they may enter into cells, even immunocells, and live, multiply, and change the memory of their host-cells. Some cancers and AIDS and the severe complication of opportunistic infections of these diseases are examples of such microbial (mis-)behavior.

The pathogenesis of a disease is thus dependent on how well the immune system is functioning and normally it is in perfect balance for its tasks. Besides the microbial interference, drugs are the main cause of temporary and permanent suppression of the immune defence and much attention in pharmaceutical research is put in this direction.

### Summary of the invention

The optimal drug would be a drug that acts in harmony and symbiotic interplay with the immune defence system and with a targeting at causes and symptoms without adverse reactions. The present invention provides novel pharmaceutical uses and novel pharmaceutical compositions which seem to have said properties. The means by which this is achieved involves the use of one or more proteolytic enzymes which have been isolated from antarctic krill (Euphasia superba). As mentioned above, such enzymes are known as such, and have also been suggested for a few pharmaceutical uses. The results obtained when using said krill enzymes for the herein disclosed purposes could not be expected, and the results obtained are, indeed, unexpected. The means by which these results are achieved will be explained in the following Examples and the appended claims.

### Preparation Example

As mentioned above, many procedures for isolating proteolytic enzymes from krill are already known, so the following is only one of many possible ways of preparing enzyme preparations which can be used according to the invention.

Deep frozen white Krill, Euphasia Superba, was thawed and homogenized. Distilled water was added at ratio of 1:1 (w/v) and 0.02% sodium azide and left for 6 hours at +4°C. The water phase was collected together with the centrifugate from meat/shell pieces, 9000 rpm for 40 minutes. The water phase was defatted With ethyl acetate at +4°C over night. The lower water phase was collected and evaporated for several hours.

Saturated ammonium sulphate solution was added to 60% saturation. The obtained precipitate was centrifugated at 9000 rpm for 40 minutes and then dissolved in 0.05M phosphate buffer, pH 7.4, 0.05M sodium chloride (PBS) and dialyzed against PBS, "crude extract".

The crude extract was applied to a Sephacryl 200 (Pharmacia, Sweden) column and protein fractions were collected at 280 nm absorbance and assayed for proteolytic activity. Active fractions were pooled and lyophilized. The combined active fractions will in the following interchangeably be called "Multi-Protein", "Multi-Enzyme", PHIM or PHIM 106.

The molecular weight range of isolated enzymes was determined by SDS-PAGE PAA4/30 (Pharmacia, Sweden). Minimum 6 bands were shown within the range of 18,000-40,000, specifically 24,000-34,000.

One fraction from the gel chromotography above showed both endo- and exopeptidase activites and the fraction was applied for further separation. It was shown that this fraction contained a possible single enzyme with a molecular weight of 26,000-32,000. It will in the following be called "Single-Protein", or "Single-Enzyme".

Neither the crude extract, nor the Multi-Protein, nor the Single-Protein showed any bacteriocidal effects in vitro, but possibly a bacteriostatic effect.

The normal temperature range of the enzymes in living shrimps is -5 to -2°C. It is surprising to find that temperature optimum for the enzymes is +55°C at neutral pH and some enzymes are stable at 100°C for 1 hour.

### Various characteristics of PHIM

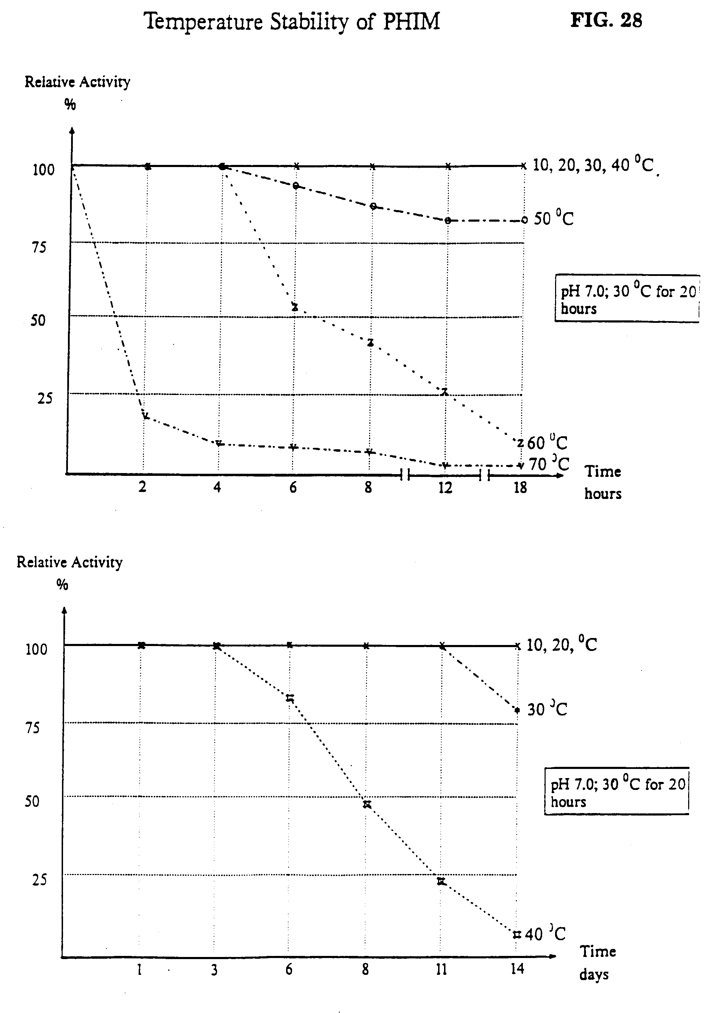

In Figure 28 the temperature stability of PHIM, as prepared above, is shown, i.e. the percentage relative activity of PHIM (activity expressed as digested area of bovine casein; BioRad Protease Substrate Tablets; 30°C for 20 hours) as a function of time at the temperatures 10, 20, 30, 40, 50, 60 and 70°C. At 70°C the activity has decreased to below 25% after 2 hours whereas this takes at least 12 hours at 60°C. At 40°C a decrease of activity below 25% is seen after 11 days whereas the activity of PHIM at 30°C after 11 days is still about 100%.

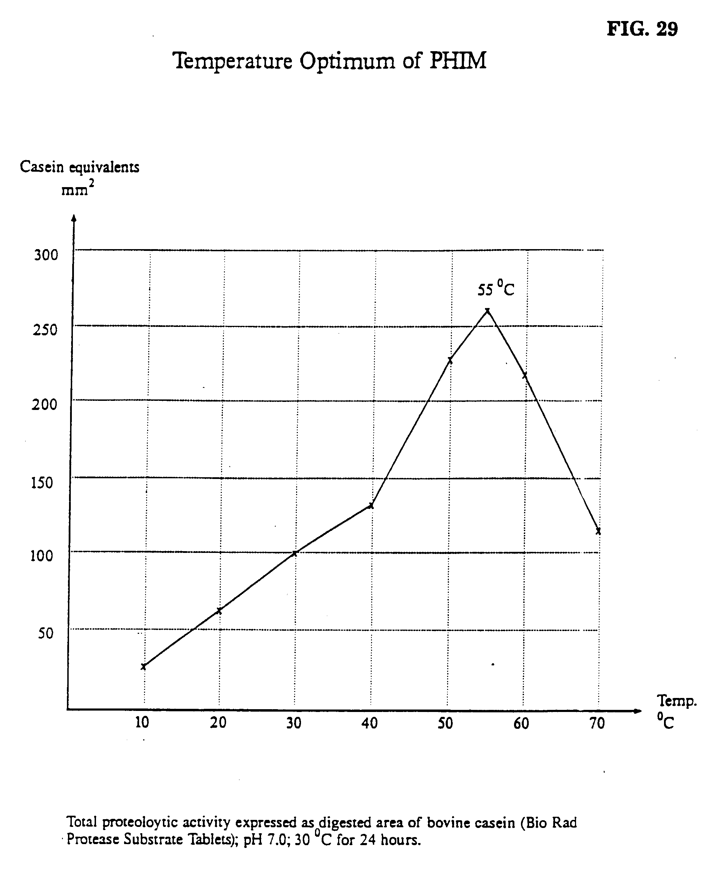

In Figure 29 the temperature optimum is shown, i.e. the total proteolytic activity of PHIM (expressed in Casein equivalents in mm² as digested area of bovine casein; BioRad Protease Substrate Tablets; pH 7.0; 30°C for 24 hours) as a function of temperature. It is interesting to note that the optimum activity is obtained at 55°C.

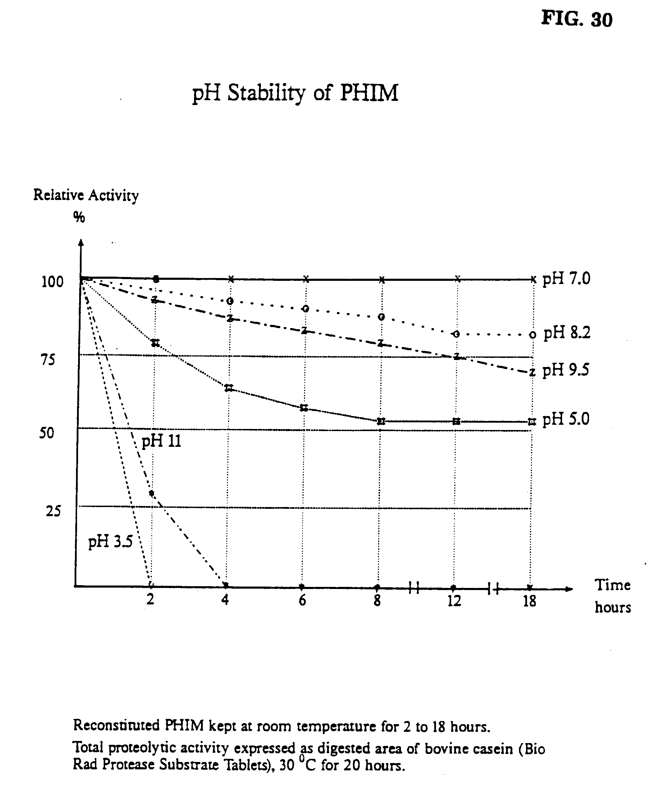

In Figure 30 the pH stability of PHIM is shown, i.e. the percentage relative activity of PHIM (activity expressed as digested area of bovine casein; BioRad Protease Substrate Tablets; 30°C for 20 hours) as a function of time at different pH valves. Reconstituted PHIM was kept at room temperature for 2 to 18 hours. At pH 3.5 and pH 11 there is a rather rapid decrease of activity already after a few hours whereas between pH 7.0 and pH 9.5 the activity is still above about 70% after 18 hours.

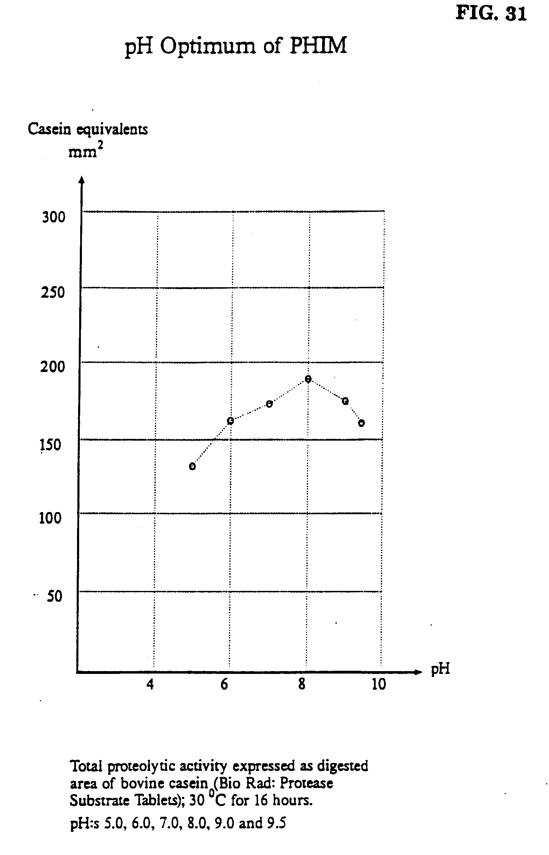

In Figure 31 the pH optimum of PHIM is shown, i.e. the total proteolytic activity of PHIM (expressed in Casein equivalents in mm² as digested area of bovine casein, BioRad Protease Substrate Tablets; 30°C for 16 hours) as a function of pH. It is noted that there is an optimum proteolytic activity at about pH 8.

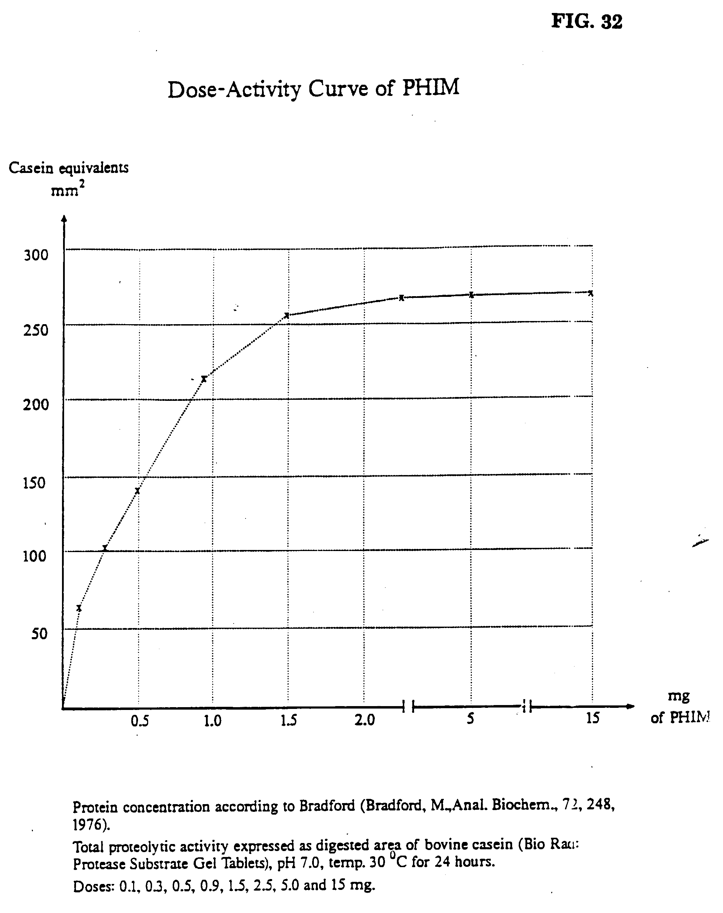

In Figure 32 an in-vitro Dose-Activity curve of PHIM is shown, i.e. the total proteolytic activity of PHIM (expressed in Casein equivalents in mm² as digested area of bovine casein; BioRad Protease Substrate Gel Tablets; pH 7.0; 30°C for 24 hours). The protein concentration was measured according to Bradford, M., Anal. Biochem. 72, 248, 1976. The activity rapidly increases with increased doses of PHIM and reaches a maximum at about 1.5 mg of PHIM, the activity still having about the same value at 15 mg of PHIM.

### Clinical Examples

To illustrate the therapeutical effect obtained according to the invention, possible limitations and drawbacks in clinical practice, the enzymes and enzyme mixtures according to the invention were tested clinically for i.a. the following indications:
- Infections
- Inflammations
- Dead and divergent cells
- Opportunistic infections
- Eye diseases
- Pain
- Cancer.

All studies were designed as pilot studies on small numbers of patients for the respective indication. To investigate the aim of study mostly topical wounds and affected sites were selected where visual inspection of clinical parameters could be performed.

In most of the cases the keyword for patient selection was "otherwise healthy patient", i.e. the focusing was made on the primary clinical problem situation and obvious underlying system factors were excluded to enable a good interpretation of results. A certain variety of general conditions and systemic factors are naturally included in this patient material since patients from the age of 20 to 85 years participated and indications from ambulatory "light" inflammation to hospitalized bed sore were included. Each test group for the respective indication constituted a rather homogeneous group regarding general conditions and underlying systemic factors.

### Brief description of the drawings

**Figures 1-4** are diagrams showing average exudation ratios, average erythema scoring, average swelling/oedema scoring and average pain for treatment of post-operative surgical wounds with Single- and Multi-Enzymes according to the invention.

**Figures 5 and 6** are diagrams showing average pain relief and inflammation scoring for 7 days' treatment of painful gum infections with Multi-Enzymes according to the invention.

**Figures 7 and 8** are diagrams showing erythema/swelling and pain sensation scoring for nine days' treatment of viral infections in the upper airways with Multi-Enzyme according to the invention.

**Figures 9 and 10** are diagrams showing average time between urinations and pain relief scoring respectively for four days' treatment of urinary bladder and urethrea infections with Multi-Enzyme according to the invention.

**Figure 11** is a diagram showing average pain relief over seven days' treatment of acute arthritis in race horses with Multi-Enzyme according to the invention.

**Figure 12** is a diagram showing the decomposing efficiency of Single-Enzyme on necrotic post-operative wounds.

**Figure 13** shows the Dose-Response curve of the Multi-Enzyme (PHIM) for the indication burns.

**Figure 14** shows Dose-Range intervals for the Multi-Enzyme (PHIM) for various indications.

**Figure 15** shows the maximal accumulated amount per kg body weight of Multi-Enzyme (PHIM) to reach a cure for various indications.

**Figure 16** shows the minimal accumulated amount per kg body weight of Multi-Enzyme (PHIM) to reach a cure for various indications.

**Figure 17** is a bar chart illustrating the effect of tumour treatment with a single injection (IT, IP and SC) of PHIM 106, compared with the control group.

**Figure 18** is similar to Fig. 17, but shows the result of repeated injection of PHIM 106 subcutaneously.

**Figure 19** is a bar chart illustrating the tumour volume for administration of PHIM 106 in varying doses and compared to the control.

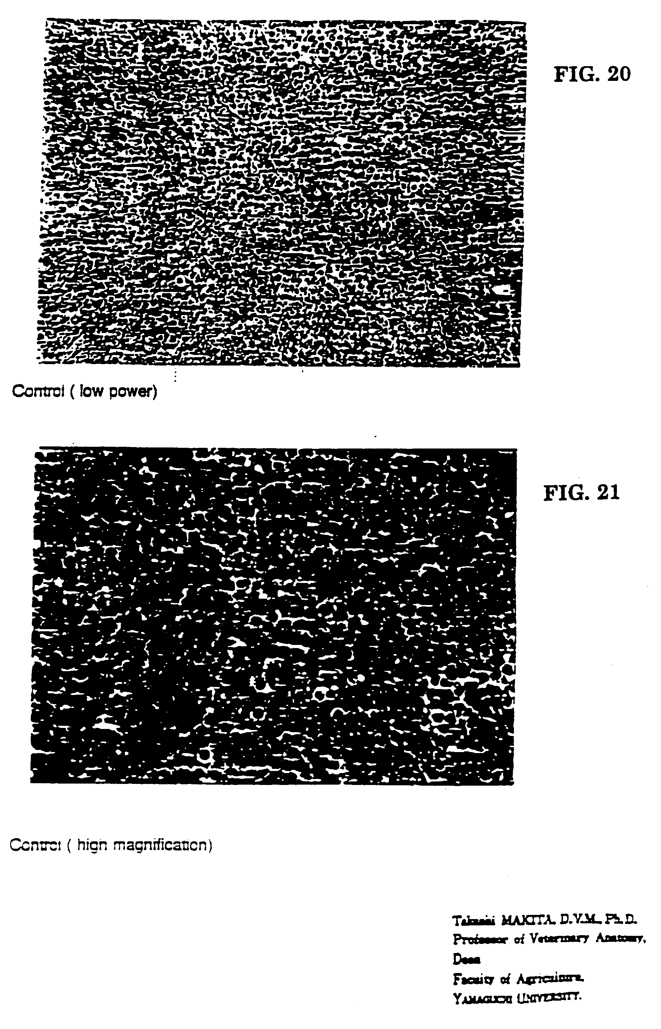

**Figures 20 and 21** show histopathological sections of control rats in two different degrees of magnification.

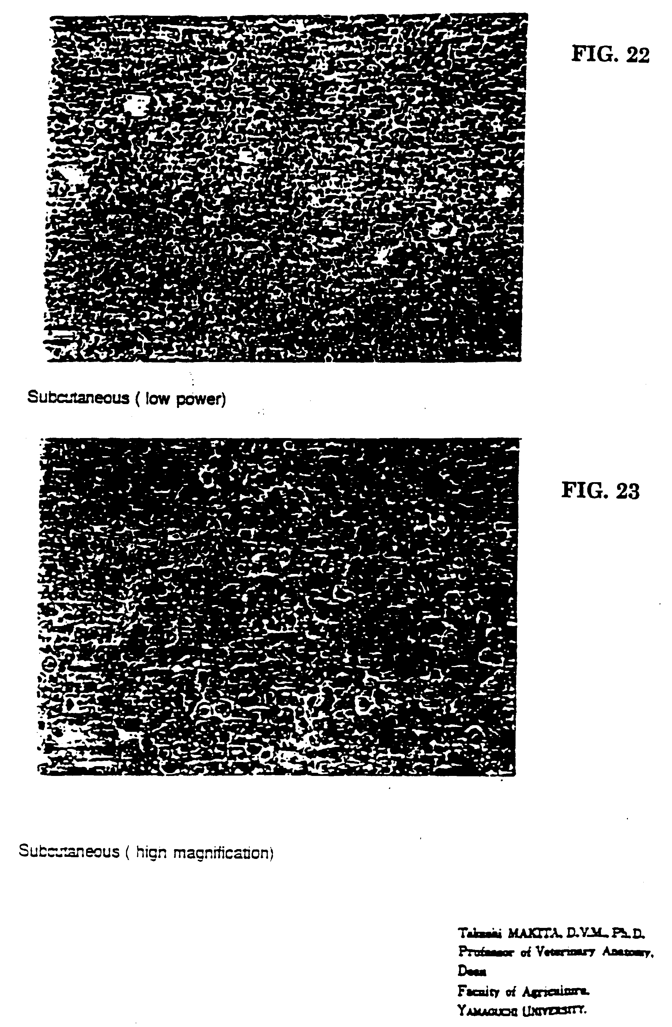

**Figures 22 and 23** show sections corresponding to Figs. 20 and 21 but for rats treated according to the invention.

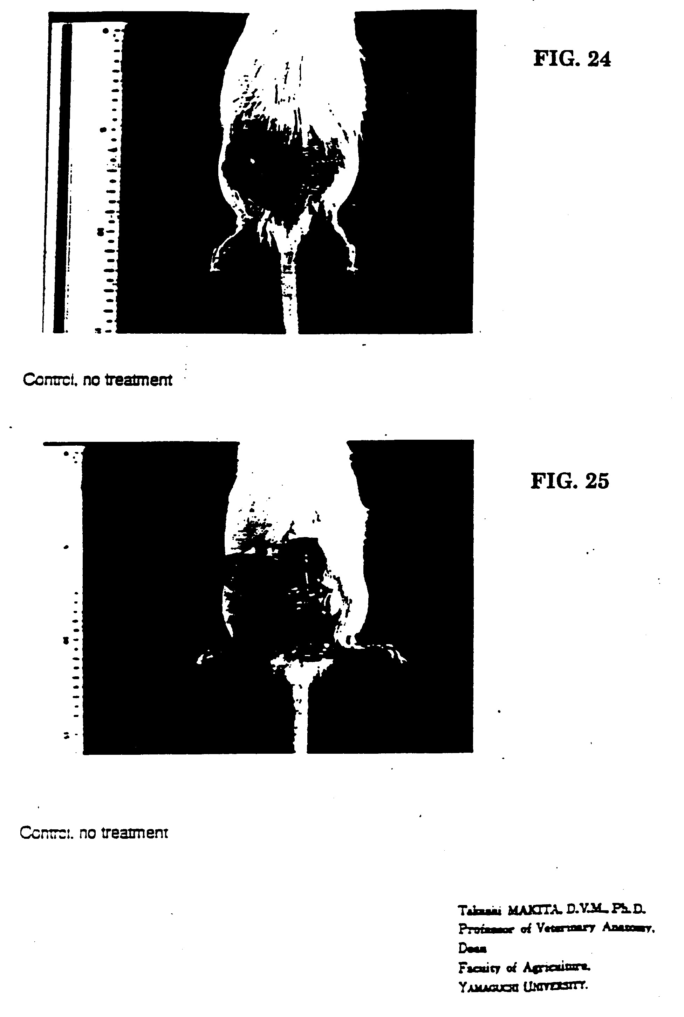

**Figures 24 and 25** are photos showing the tumours on an untreated control rat, and

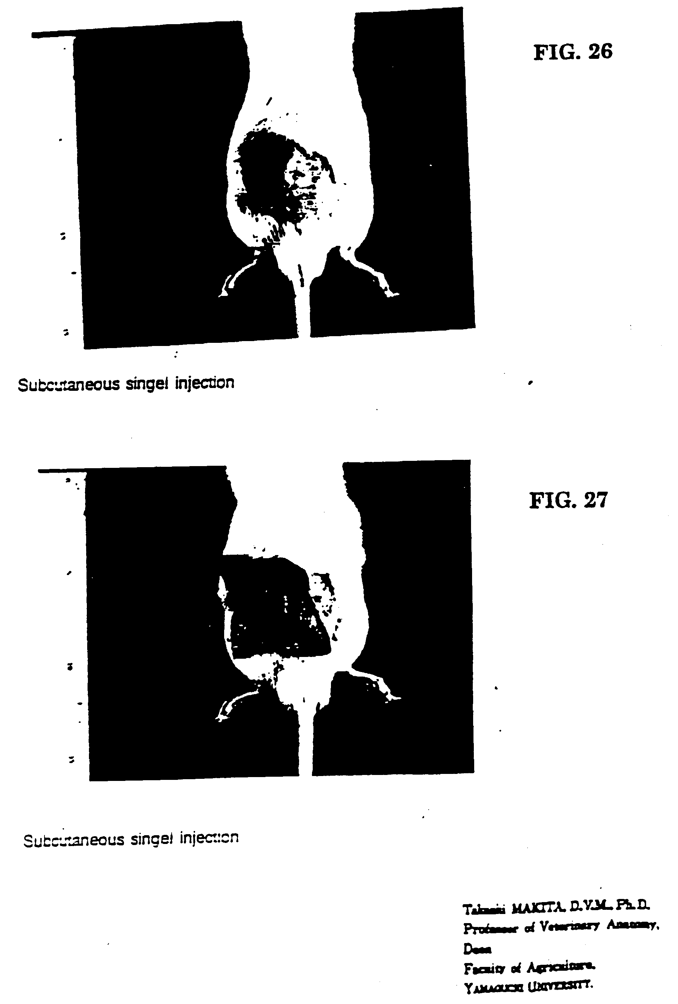

**Figures 26 and 27** show corresponding photos of a rat treated with a single subcutaneous injection according to the invention.

**Figure 28** shows the temperature stability of PHIM, i.e. the percentage relative activity of PHIM versus the time at different temperatures.

**Figure 29** shows the temperature optimum of PHIM, i.e. the total proteolytic activity as a function of the temperature.

**Figure 30** shows the pH stability of PHIM, i.e. the percentage relative activity versus the time at different pH values.

**Figure 31** shows the pH optimum of PHIM, i.e. the total proteolytic activity as a function of the pH.

**Figure 32** shows a Dose-Activity curve of PHIM, i.e. the total proteolytic activity as a function of the dose of PHIM.

### Clinical Examples 1 to 10 - Infections

Infections and inflammations most often occur together and they show similar clinical signs, i.e. erythema, swelling, heat, oedema, exudation, pus, pain. necrotic tissues, and sometimes smell. These clinical signs were followed and recorded to evaluate the course of treatment and efficacies of the Single-Protein and Multi-Protein preparations according to the invention, as described above.

### Example 1 - Post-operative surgical wounds

Totally 40 patients were included in this study and they were divided into two groups of 20 patients each, representing 41 post-op. abdominal (34) and thoracic(7) wounds. Single-Enzyme, 3 Casein-Units/ml, resp. Multi-Enzyme, 5 Casein- units/ml, preparations from Krill were tested in each group. Lyophilized white powder without preservatives or anti-microbial additives. To be reconstituted in 5 ml saline.

The patient material had an average age of 52±16 years, including 28 males and 12 females. The anti-microbial actions of the preparations were very good to excellent regarding clinical effects and efficiency. Within 5 days all infections were brought to a subclinical level and all obvious signs of clinical infections were gone. No notable difference between the two preparations could be observed. There were no obvious or suspected adverse reactions observed.

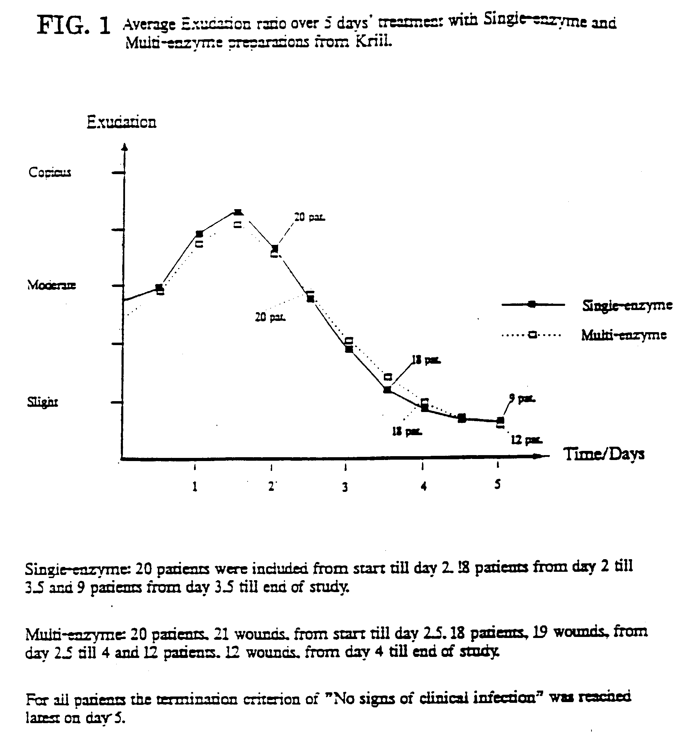

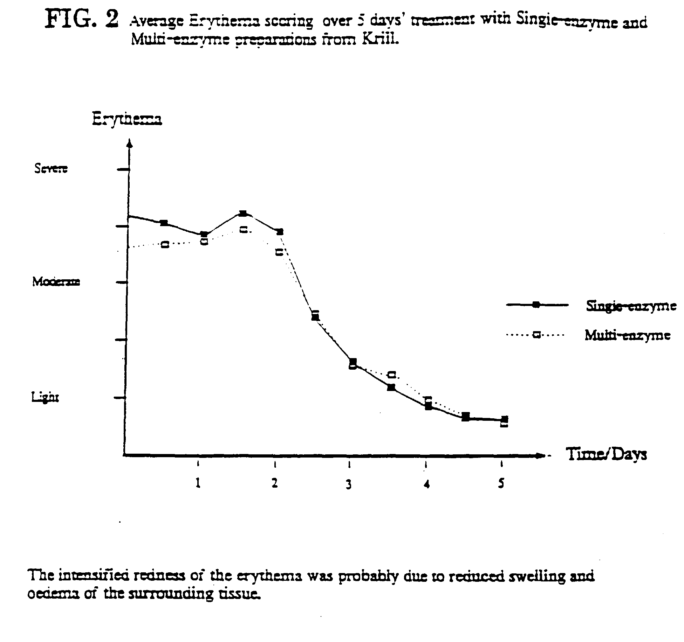

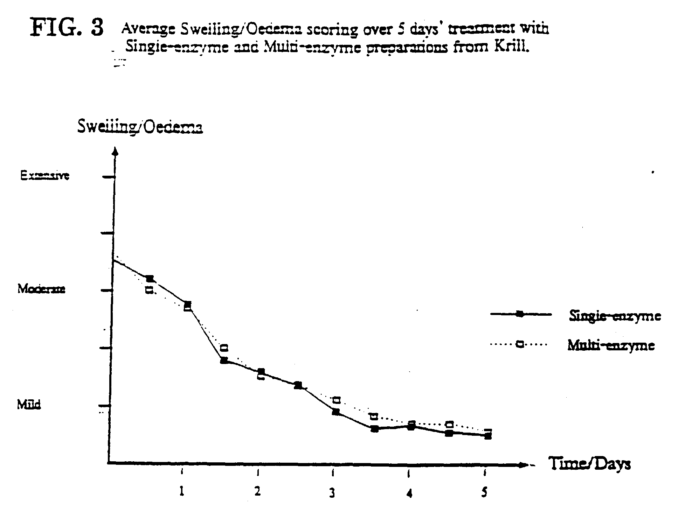

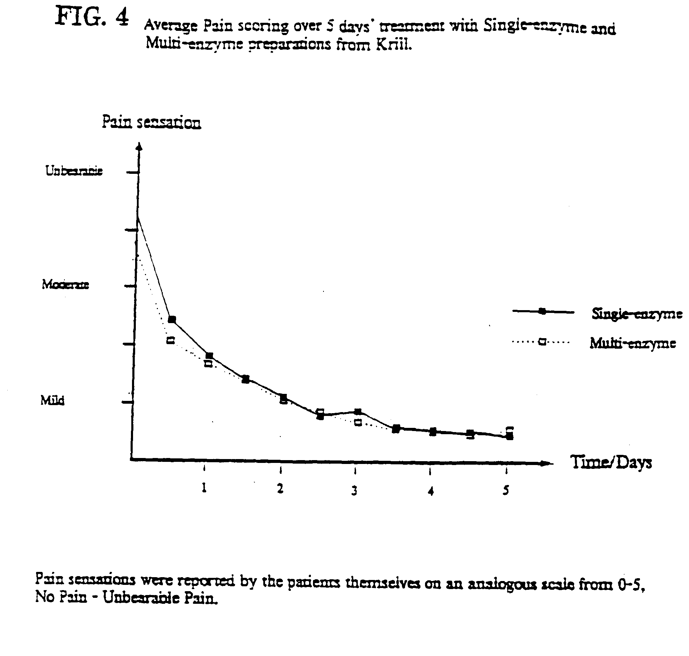

The results are summarized in Figures 1-4.

A suitable dose range for this indication is 0.01 to 100, preferably 1 to 25 mg per 100 cm².

### Example 2 - Small size burns

11 patients with smaller full thickness burns infected by S. aureus and P. aeruginosa not responding to antibiotics and silverdiazine cream were included in this study. 5 patients were treated with Single-Enzyme hydrocolloid cream, 3 Casein-Units/ml, and 6 patients with Multi-Enzyme solution, 5 Casein-Units/ml. Lyophilized white powder without preservatives or anti-microbial additives. Multi-Enzyme preparation: 1 ampoule to be reconstituted in 5 ml saline to a final concentration of 5 Casein-Units/ml solution. Single-Enzyme preparation 1 ampoule to be mixed with 5 ml of hydrocolloid gel to a final concentration of 3 Casein-Units/ml gel.

A suitable dose range for this indication is 0.01 to 100, preferably 1 to 25 mg per 100 cm².

All wounds were completely free from all signs of infection within 5 days' treatment which was confirmed by MO-cultivation. Necrotic tissue, pus and fibrinous fibrils in the granulation tissue were effectively decomposed by both preparation and no perceptible difference in efficacy between the preparations could be observed. No adverse reactions could be noted from the preparations.

The test results are summarized in Table 1.

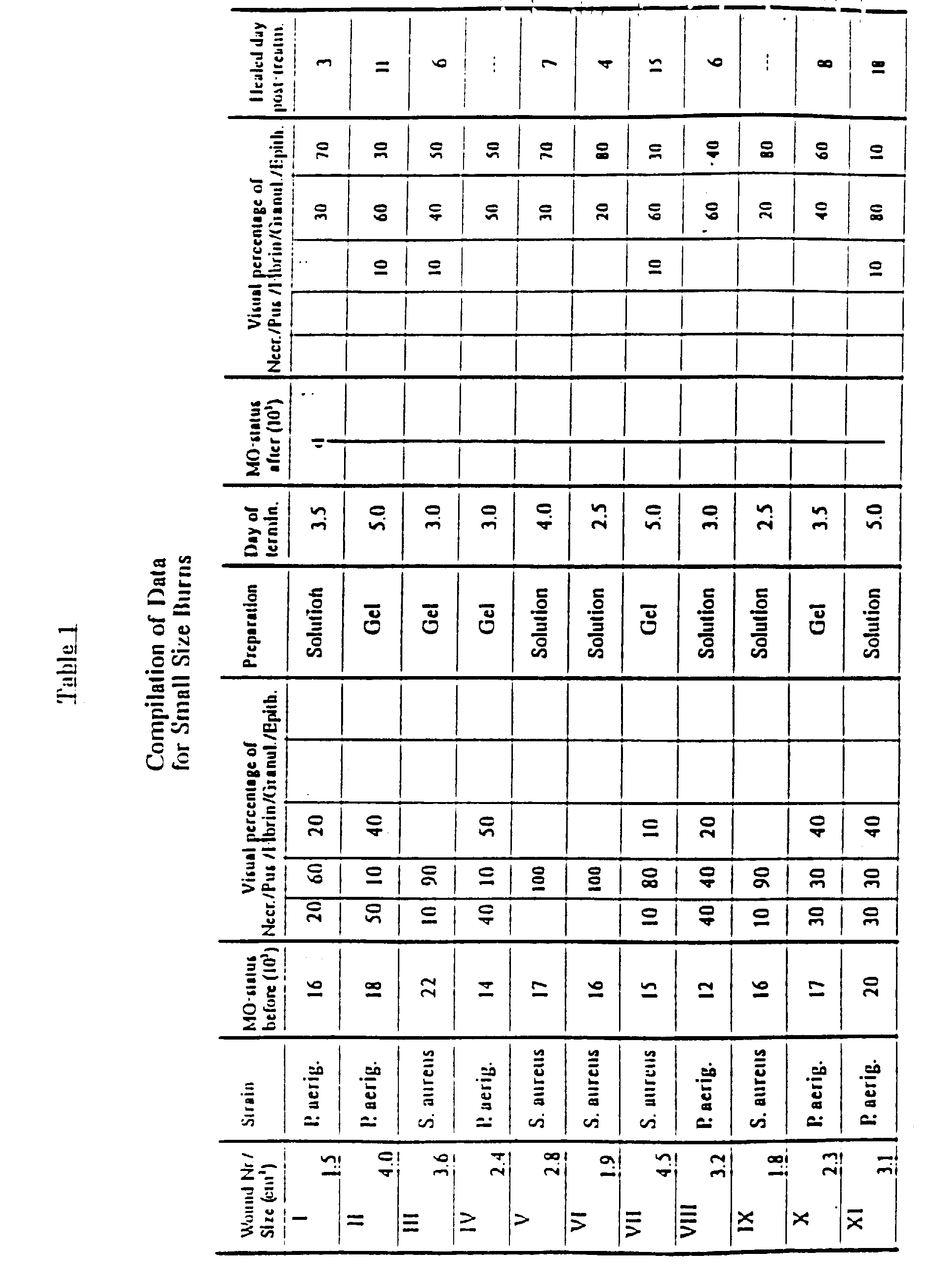

### Example 3 - Painful gum infections

22 patients with acute or chronic gum infections/inflammations were included in this study. Lyophilized white powder without preservatives or anti-microbial additives. Multi-Enzyme preparation: 1 ampoule to be reconstituted in 5 ml saline to a final concentration of 5 Casein-Units/ml solution.

Three times a day, morning, mid-day and evening, an ampoule was reconstituted in 5 ml tap water and the mouth cavity was rinsed for 5 minutes. No eating and drinking within 2 hours after treatment was allowed. The treatment went on for 7 days independent of results.

Pain relief was reported after 20 minutes' to 12 hours' treatment. Infections and inflammations vanished within 4 days and did not reoccur during the follow-up period of 3 weeks. No adverse reactions were reported.

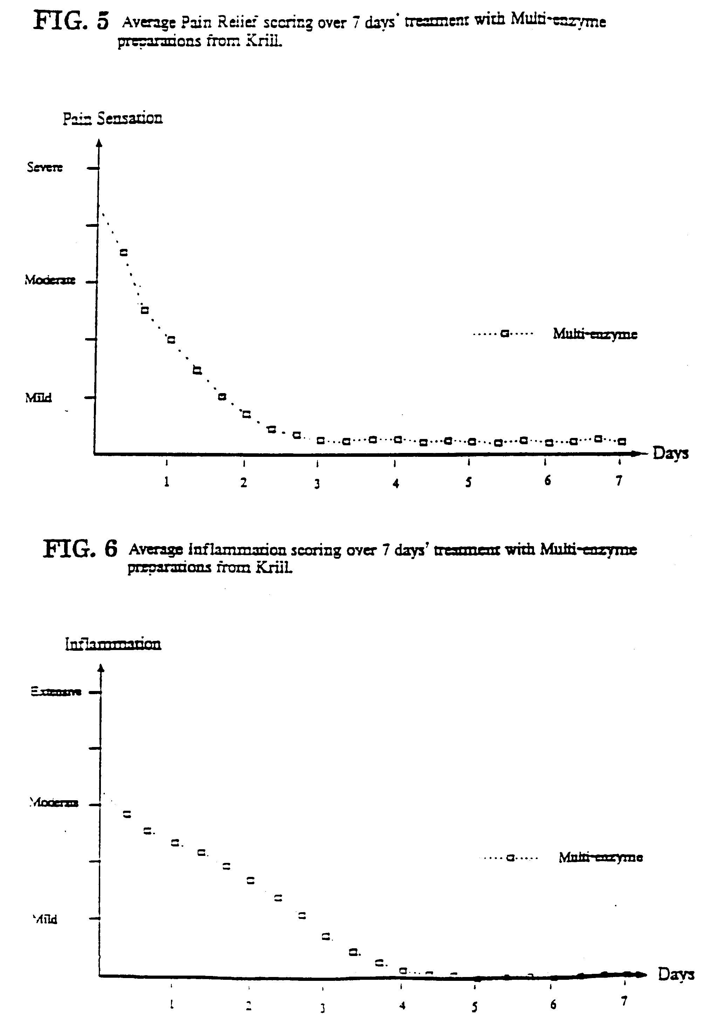

The results are summarized in Figures 5 and 6.

A suitable dose range for this indication is 0.1 to 100, preferably 1 to 35 mg per treatment.

### Example 4 - Viral infections in the upper airways

11 patients with Influenza Virus infections and secondary bacterial infections in the upper airways (e.g. sinusitis) were included in this study.

Viral infections in the airways cause harm to the cilia and an inflammatory reaction is initiated with erythema, swelling and increased mucus-secretion. The harmed cilia can no longer wipe away inhaled bacteria and bacterial infections often become secondary complications to viral infections.

Other typical symptoms of viral infections are general state of illness. strong sense of fatigue, fever and general pain.

Viruses are dependent on host-cells for their survival and multiplication and it is a difficult task to kill a virus without harming/killing the host-cell.

Multi-Enzyme preparation: Lyophilized white powder without preservatives or anti-microbial additives was used. One ampoule to be reconstituted in 5 ml of saline to a final concentration of 5 Casein-Units per ml.

Approximately 0,25 ml of the test solution was sprayed in each nostril and the mouth-cavity was rinsed for 5 minutes with approx. 4.5 ml. The procedure was repeated three times daily and clinical parameters, erythema, swelling, mucus-secretion, pain and adverse reactions were recorded once daily.

The treatment was terminated when all signs of infection were gone but for no longer than 10 days.

8 patients were free from symptoms after 6 days' treatment and the remaining three patients after 9 days.

Pain relief was experienced in all patients and occurred within 2 hours to two days. Sputum became clear within three days in patients with purulent discharges. Erythema and swelling disappeared within 4 days.

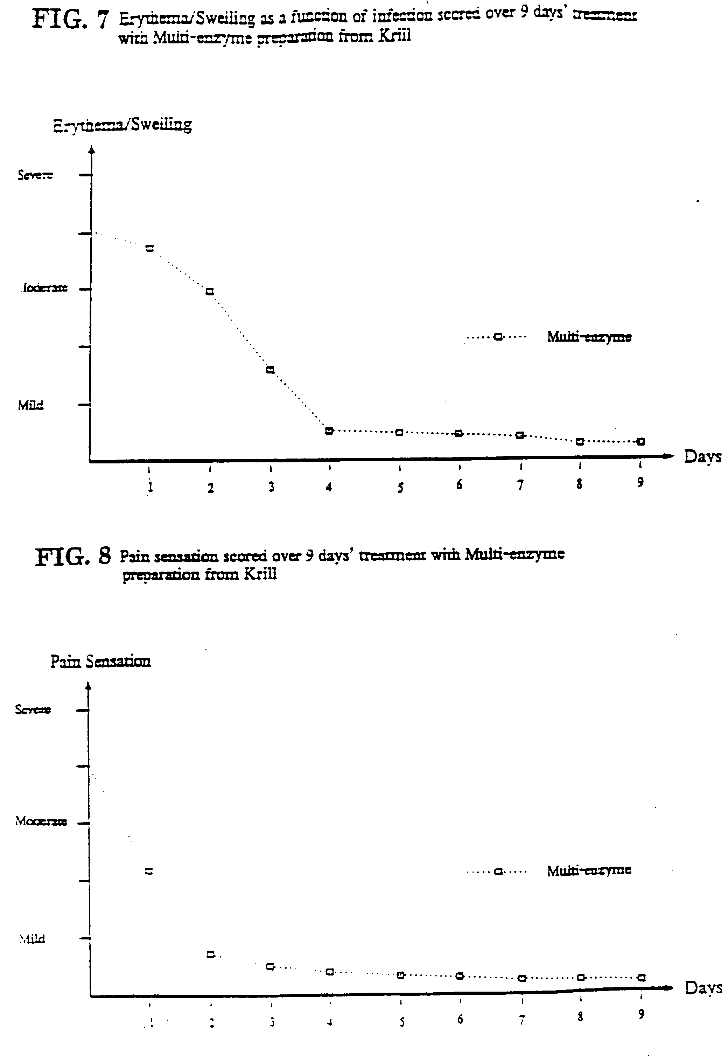

The results are summarized in Figures 7 and 8. No adverse reactions were observed.

A suitable dose range for this indication is 0.1 to 100, preferably 1 to 35 mg per treatment.

### Example 5 - Herpes Simplex infection in the mouth cavity

8 patients with relapsed Herpes Simplex blisters in mouth cavity were treated twice daily with mouth-wash. Lyophilized white powder without preservatives or anti-microbial additives. Multi-Enzyme: 1 ampoule to be reconstituted in 5 ml of saline to a final concentration of 5 Casein-Units/ml.

An ampoule was prepared before each treatment and the mouth cavity was rinsed for 5 minutes with test solution. No eating or drinking was allowed within 2 hours after each treatment. The procedure was repeated twice daily and clinical parameters, erythema, swelling, pain and adverse reactions were recorded once daily.

The treatment was terminated when all signs of infection were gone but for no longer than 10 days.

Pain relief was experienced within 2 hours after the first treatment and in some patients the pain recurred between treatments during the first two days but never thereafter.

After 5 days all patients were free from symptoms and all blisters had healed. No adverse reactions were observed.

It appears from the above Examples 1 to 5 that bacterial, viral and fungal infections follow very similar courses of progress and all signs of infections and inflammations have vanished within 3-6 days. Even drug-resistant bacterial strains, such as S. aureus and P. aeruginosa in burns, and intra-cellular virus, such as Herpes Simplex, follow the same pattern. Gum infections and viral infections in the upper airways were also effectively treated only with rinsing of the mouth-cavity for totally 15 minutes per day for 5-7 days. The short effective time of action and the possible rinsing-away effect from saliva supports the hypothesis that the proteins from the test preparations have an ability to adhere to cells or surfaces and to perform actions for much longer time than the actual treatment sequence. The identical observation was made in patients with extreme lacrimal secretion in eye infections.

A suitable dose range for this indication is 0.1 to 100, preferably 1 to 35 mg per treatment.

### Example 6 - Infected decubitus ulcera

14 elderly patients with totally 18 decubitus on heels and lower back were included in this study. Lyophilized white powder without preservatives or anti-microbial additives. Multi-Enzyme preparation: 1 ampoule to be reconstituted in 5 ml saline to a final concentration of 5 Casein-Units/ml solution.

Ulcera were rinsed thoroughly with saline and emptied as far as possible before instillation of 5 ml Multi-Enzyme Solution into the ulcer and covered with semi- occlusive dressing. The procedure was repeated twice daily for 7 days and ulcera were inspected for inflammation, erythema, heat, swelling, necrotic tissue, pus, pain and possible adverse reactions.

Infections were gone within 4 days' treatment. 6 wounds healed completely within 7 days and totally 11 within 14 days. 7 wounds did not heal due to the general conditions of the patients but the wounds showed some progress. No adverse reactions were observed.

A suitable dose range for this indication is 0.1 to 100, preferably 1 to 25 mg per 100 cm².

### Example 7 - Fistulae infections

The purpose of the study was to investigate the anti-microbial and decomposition efficacies and usefulness of Single-Protein and Multi-Protein preparations from Krill on anal fistulae. Lyophilized white powder without preservatives or anti-microbial additives.
Single-Protein with both endo- and exopeptidase activities: 1 ampoule to be reconstituted in 5 ml of hydrogel to a final concentration of 3 Casein-Units/ml. Multi-Protein with a mixture of endo- and exopeptidase activities: 1 ampoule to be reconstituted in 5 ml of hydrogel to a final concentration of 5 Casein-Units/ml.

The fistulae were rinsed with sterile solution and emptied as far as possible before instillation of Single-Protein Gel and Multi-Protein Gel, respectively. The procedure was repeated once daily and patients were inspected for erythema, heat, sweiling, pus, pain and adverse reactions.

The treatment was terminated when all signs of infection and inflammation were gone but for no longer than 10 days.

2 + 2 patients with anal fistulae, with no passages to rectum, were included in this study and treated with Single-Protein and Multi-Protein gel preparations, respectively.

Total pain relief was reported within 48 hours and all signs of infections and inflammations were gone after 4 days.

All fistulae were healed on Day 6 to Day 9 and no recurrence was reported within 6 months' follow-up. No adverse reactions were observed.

It appears from the above Examples that complicated infections that generally are regarded by expertise to be very difficult to treat, such as anal fistulae and decubitus ulcera, were successfully treated and led to final healing in most of the cases within the time-frame of the respective study.

A suitable dose range for this indication is 0.01 to 100, preferably 1-25 mg per 100 cm².

### Example 8 - Eve infections

15 patients with purulent eye infections were treated twice daily with eye-drops of Multi-Enzyme preparation from Krill. (Lyophilized white powder without preservatives or anti-microbial additives. One ampoule of Multi-Enzyme to be reconstituted in 25 ml Water for Injection to a final concentration of 1 Casein-Unit/ml.) The infected eye was dropped morning and evening with two drops, approx. 0.4 ml, of the test solution. At each application the eye was inspected for erythema, swelling, pus, lacrimal secretion and possible adverse reactions.

The treatment was terminated when all signs of infection were gone but for no longer than 10 days.

All patients were free from infections within 3 days' treatment. Erythema and swelling around the eyes faded away within 2 days and excess lacrimal secretion ceased within 2 days as well. Instantly after the first application all patients experienced a smoothing feeling in the infected eye and irritation and tenderness around the eyes disappeared within a few minutes. No adverse reactions were reported.

It appears from the above Example that eye infections responded quickest to the treatment even though very low concentrations were used, 0.4 Casein-Units/treatment.

A suitable dose range for this indication is 0.01 to 50, preferably 0.1 to 5 mg per treatment.

### Example 9 - Prophylactic treatment of post-op, wounds

Single-Enzyme and Multi-Enzyme preparations from Krill were tested against a sterile 0.9% NaCl control solution, as a prophylactic anti-microbial rinsing solution on post-op. wounds, totally 60 patients with 20 patients in each group.

Non-bleeding post-op. wounds, first treatment 6-12 hours post-op., were redressed twice daily with resp. solution. The wounds were rinsed thoroughly with resp. solution and covered with sterile gauze under semi-occlusive dressing. At each redressing the wounds were inspected for infection, inflammation, erythema, swelling, heat, necrotic tissue, fibrin, pus, bleeding, pain and possible adverse reactions.

Treatment was terminated when wounds were healed, >90% epithelialization, or when test or control treatments failed.

No post-op. clinical infections occurred in the groups treated with Single-Enzyme or Multi-Enzyme solutions nor were acute inflammation or erythema observed in any of the patients in these two groups. 18 wounds were healed within 10 days' treatment. No adverse reactions were observed.

In the control group 4 patients developed severe invasive infections and additional 2 patients had acute inflammation. Erythema, swelling and pain were frequent observations in this group. 14 wounds were healed within 10 days' treatment.

A suitable dose range for this indication is 0.01 to 100, preferably 1 to 25 mg per 100 cm².

### Example 10 - Urinary bladder and urethra infections

12 patients, only females, with painful urinary infections were included in this study. Lyophiiized white powder without preservatives or anti-microbial additives. Multi-Enzyme preparation: 6 ampoules to be reconstituted in 50 ml saline to a final concentration of 3 Casein-Units/ml solution.

First discharge of urine from all patients was very turbid and second discharge was clear to weakly turbid.

Pain relief was instant and improved ability to retain urine was obvious are two days treatments. MO-samples confirmed no bacteria after 4 days' treatments and all treatments were terminated after 4 days. No adverse reactions were observed.

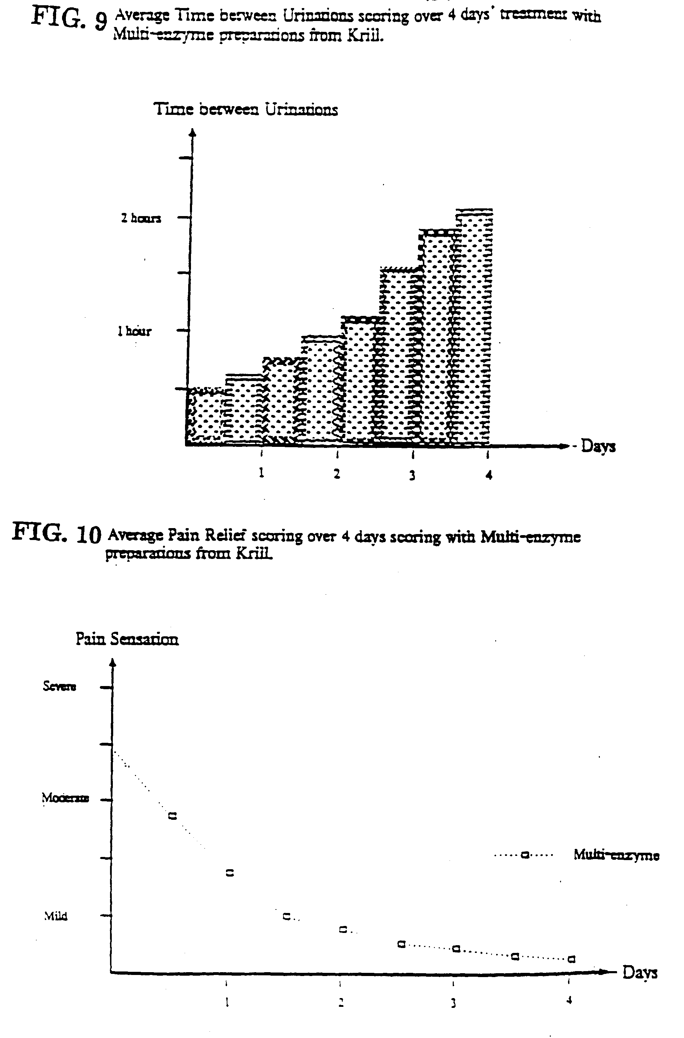

The results are summarized in Figures 9 and 10. The average MO-status values are shown in Table 2.

**Table 2**

| Average MO-status on 4 days treatment with Krill Multi-Enzyme preparation | | |
|---|---|---|
| MO-status Before treatment | MO-status Day 2 | MO-status Day 4 |
| 1.4 x 10⁴ | 4.3 x 10³ | <1.0 x 10² |
| 100% | 31% | <1% |

It appears from the above Example that urinary bladder and urethra infections responded very well to instillation treatment, twice daily. After 2 days' treatment the acute signs of infection and inflammation had disappeared and after 4 days' treatment all patients were free from symptoms.

A suitable dose range for this indication is 0.1 to 200, preferably 1 to 50 mg per treatment.

### Clinical Examples - Inflammations

Inflammations is a common feature in most of the studies and the reduction of inflammation was similar in all indications. In 3 to 4 days inflammations were reduced to acceptable levels for a sound healing process. In the prophylactic study of post-op. wounds (Example 9) no inflammation occurred in the test groups. Cf. also Example 3 (acute or chronic gum infections/inflammations).

### Example 11 - Inflamed horse joints

16 lame trotters with inflamed foreleg knees were included in this study. Lyophilized white powder without preservatives or anti-microbial additives. Multi-Enzyme preparation: 1 ampoules to be reconstituted in 10 ml Water for Injection to a final concentration of 2.5 Casein-Units/ml solution.

2 ml of Test Solution were injected into the painful joint on Day 1, 2, 4 and 7. After injections the horses were observed for any instant adverse reactions or deteriorations in general conditions. The clinical parameters were observed once daily.

9 cases responded with a quick pain relief, 30 minutes to 2 hours after the first treatment, 4 more cases within 6 hours and 2 more cases within 2 hours after the second injection. 1 case showed pain relief after the third injection and this case had an extreme swelling over the knee.

Heat and swelling were reduced rapidly and no signs of inflammation could be observed after two days for 15 of the included cases. No adverse reactions were observed.

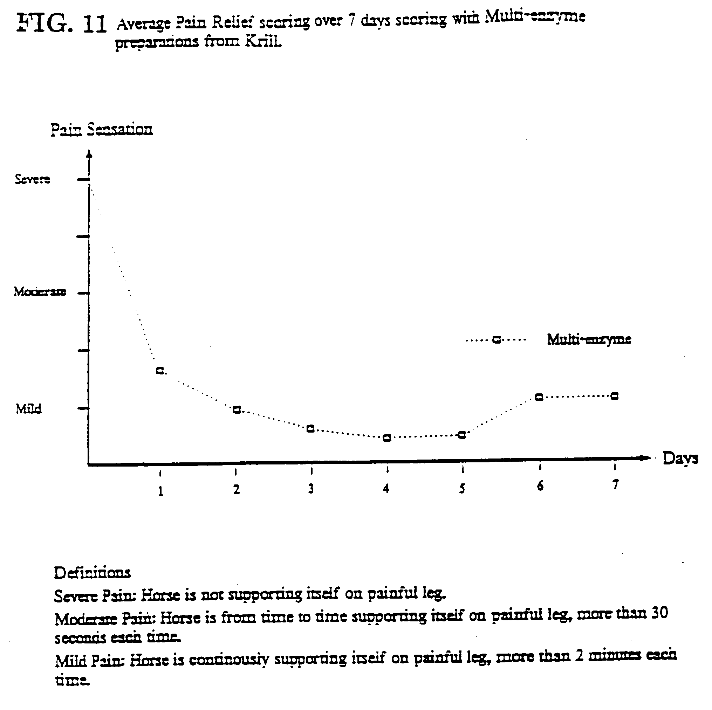

The results are summarized in Figure 11.

It appears from the above Example that acute arthritis in race horses can be treated with intraarticular injections in a sequence treatment of 4 injections over 7 days. The clinical signs of inflammation were rapidly reduced and heat and swelling faded away within 2 days. One week post-treatment the horses were back on easy training.

A suitable dose range for this indication is 0.1 to 200, preferably 1 to 50 mg per treatment.

### Clinical Examples 12-16 - Dead and Divergent Cells

Cf. also Examples 3, 4, 5 and 10.

### Example 12 - Necrotic post-operative wounds

15 patients with necrotic wounds were treated with Single-Enzyme preparations from krill. Lyophilized white powder without preservatives or anti-microbial additives. To be reconstituted in 5 ml saline. Single-Enzyme preparation: 3 Casein-Units/ml.

Normal rinsing and wound toilet was performed prior to applying the Single-Enzyme. Twice daily an ampoule was diluted in 5 ml saline and poured onto a gauze dressing which covered the wound completely. The drained gauze was fixed to the wound by a self-adhesive semi-occlusive dressing. At every change of dressing the wound was visually inspected for erythema, oedema, bleeding, swelling, heat, exudation, pus, necrotic tissue, pain, smell, possible adverse reactions and general status of patient. The treatment was terminated when all necroses, fibrin, pus and blood clots were decomposed but for no longer than 7 days.

This patient material was heterogeneous with respect to the ethiology of the wounds, i.e. scheduled operations, traumas, burns, shots and diabetes patients. All patients were treated poly-clinically twice a day. No adverse reaction were observed from the test preparations.

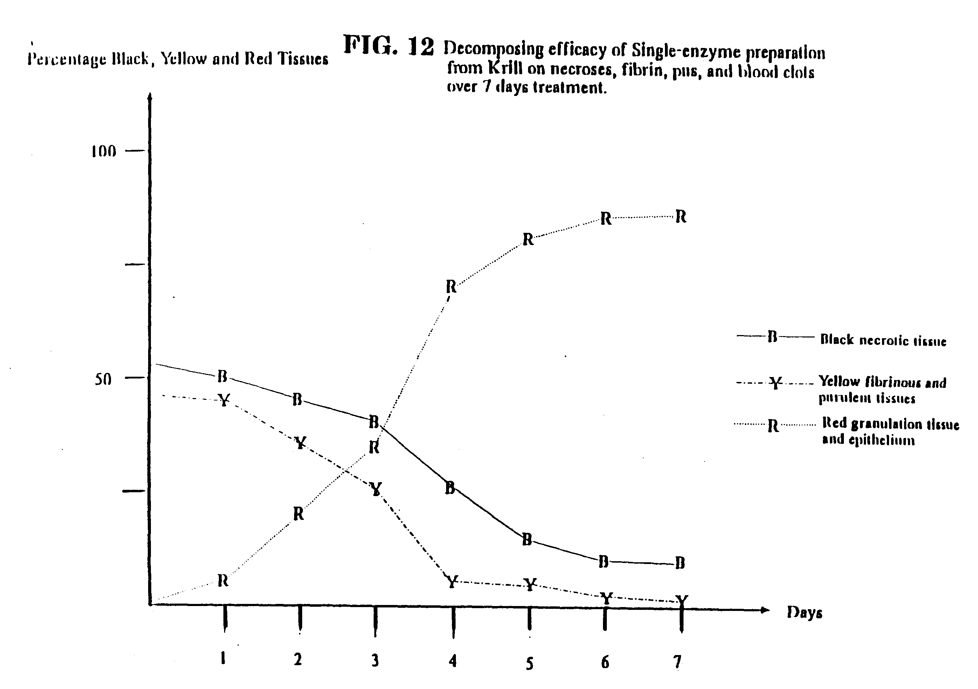

The results are summarized in Figure 12.

As can be seen from the above, necroses, fibrin, pus, blood clots, and plaques were effectively decomposed within a week and some wounds healed within a week. Burns, shots, and post-op. wounds in diabetes-patient initially showed very poor efficacy but at termination of the study, 7 days, the necroses were completely decomposed from underneath and what remained was only the top surfaces of the necroses, like a lid, and the wounds were partially healed within a week.

A suitable dose range for this indication is 0.01 to 100, preferably 1 to 25 mg per 100 cm².

### Example 13 - Decomposition of scar formation and keloids

The purpose of this study was to investigate the decomposing efficacy and usefulness of Multi-Protein preparation from Krill as an alternative to surgical revision. Lyophilized white powder without preservatives or anti-microbial additives. Multi-Protein with endo- and exopeptidase activities: 1 ampoule to be reconstituted in 5 ml Water for Injection to a final concentration of 5 Casein-Units/ml.

For every centimeter of the scar/keloid 0.2 ml of Multi-Protein solution was injected to a maximum volume of 1 ml per lesion and day. The procedure was repeated once daily and the scar/keloid was inspected for erythema, swelling, heat, bleeding, necroses and adverse reactions.

The treatment was terminated at 80% decomposition of scar/keloid but for no longer than 7 days.

Multi-Protein preparation was injected once daily into 5 facial scar formations and 3 keloid formations on hand and forearms.

Fibrinous scars were reduced to approx. 25% of their initial volumes and coilagenous keloids to approx. 70% after 7 days' treatment. No adverse reactions could be observed during this trial.

As can be seen from the above intradermal and subcutaneous scar formations and keloids were decomposed to 75% resp. 30% after 7 days' treatment with intratissual injections. In cosmetic corrections of scars and keloids the decomposition must not be too quick and seldom more than 60-80% of the scars/keloids need to be removed for best results. The skin and underlying tissues need to adapt slowly to the new situation if not other defects, such as wrinkles, shall occur.

A suitable dose range for this indication is 0.1 to 50, preferably 1 to 10 mg per treatment.

### Example 14 - Psoriasis and dry eczema

Psoriasis plaques and dry eczema plaques were extremely easily decomposed with Hydrogel test preparations (Single-Enzyme) under semi-occlusive dressings. Within 24 hours the plaques were completely gone and the sensitive skin, especially in psoriasis patients, did not show any additional inflammation, irritation, or pain. On the contrary, the affected skin areas showed less inflammation after treatment but most of all the accessibility of steroid creams was improved when plaques were gone and much better effects could be observed from those preparations.

A suitable dose range for this indication is 0.01 to 100, preferably 1 to 25 mg per 100 cm².

### Example 15 - Dental plaque in dogs

The purpose of this study was to investigate the decomposing efficacy and usefulness of Multi-Protein preparation from Krill on dental plaque in a dog model. Lyophilized white powder without preservatives or anti-microbial additives. Multi-Protein with endo- and exopeptidase activities: 1 ampoule to be reconstituted in 5 ml of saline to a final concentration of 5 Casein-Units/ml.

The content from a freshly prepared ampoule was carefully painted over teeth and gingiva. The tongue was fixated for minimum 2 minutes and food and beverage were not allowed for 2 hours post-treatment. The treatment was repeated twice daily until all plaque was completely decomposed. The dogs were inspected for status of plaque, saliva secretion and adverse reactions once daily.

8 beagles with abnormal plaque formation due to special feeding and housing were included in this study. After 4 days all signs of plaque were gone and the study was terminated. No adverse reactions could be observed.

A suitable dose range for this indication is 0.1 to 100, preferably 1 to 35 mg per treatment.

### Example 16 - Human dental plaque

The purpose of this study was to investigate the decomposing efficacy and usefulness of Multi-Protein preparation from Krill on dental plaque Lyophilized white powder without preservatives or anti-microbial additives. Multi-Protein with endo- and exopeptidase activities: 1 ampoule to be reconstituted in 5 ml of saiine to a final concentration of 5 Casein-Units/ml.

An ampoule of Multi-Protein solution was prepared before each treatment and the mouth cavity was rinsed for 5 minutes. Food and beverage were not allowed for 2 hours post-treatment. The treatment was repeated twice daily and the patients were inspected once daily for plaque, saliva secretion, dryness, and adverse reactions. The patients were not allowed to brush their teeth during ongoing study.

The treatment was terminated when all signs of plaque were gone but for no longer than 7 days.

2 hours after the first treatment all patients experienced a soft and smooth sense over the teeth and all patients believed the plaque was completely decomposed. Visual inspection showed remnants of plaque. 2 hours after the third treatment all signs of plaque were gone and treatments were terminated. No adverse reactions could be observed.

A suitable dose range for this indication is 0.1 to 100, preferably 1 to 35 mg per treatment.

### Example 17 - Opportunistic Infections

These types of infections are developed in patients with totally suppressed immune system, caused by cancer, AIDS, immune suppressing drugs, irradiation, etc. Pathogenic microbial strains, and normally non-pathogenic strains may turn pathogenic, cause severe infections that finally lead to general sepsis which is the actual cause of death of patients suffering from these conditions.

Three patients, 2 patients in uterine cancer in stage IV and 1 patient with irradiation wound, with opportunistic infections in non-healing post-op. wounds were treated twice daily with drained dressings. After 12, 14 and 17 days the wounds had healed.

A suitable dose range for this indication is 0.01 to 100, preferably 1 to 25 mg per 100 cm².

### Clinical Examples 18-19 - Eve Diseases

### Example 18 - Gray cataract on dog

The purpose of the study was to investigate the efficacy and usefulness of Single-Protein and Multi-Protein preparations from Krill on gray cataract in dog. Lyophilized white powder without preservatives or anti-microbial additives. Single-Protein with both endo- and exopeptidase activities: 1 ampoule to be reconstituted in 5 ml of Water for Injection to a final concentration of 3 Casein-Units/ml. Multi-Protein with a mixture of endo- and exopeptidase activities: 1 ampoule to be reconstituted in 5 ml of Water for Injection to a final concentration of 5 Casein-Units/ml.

The right eye was treated with 2 drops, approx. 0.4 ml, of Multi-Protein solution. The procedure was repeated once daily and the dog was inspected for changes in opacity and adverse reactions.

The treatment was terminated when the eye appeared clear by visual inspection. The left eye was treated with Single-Protein solution according to an identical procedure as for Multi-Protein solution.

The treatment was terminated when the eye appeared clear by visual inspection.

After 5 days an obvious diminishing in the opacity could be noted in the right eye and after 10 days the eye looked very clear and the treatment was terminated.

Identical observations were made and treatment was terminated after 10 days also for the left eye. No adverse reactions were observed.

A suitable dose range for this indication is 0.01 to 50, preferably 0.1 to 5 mg per treatment.

### Example 19 - Cataract of the eve

The purpose of the study was to investigate the efficacy and usefulness of multi-Protein preparation from Krill on gray cataract.

Multi-Protein with a mixture of endo- and exopeptidase activities, 2.5 Casein-units/ml, was dropped into the right eye every 3 days.

An old lady in her 80's with day/night vision on one eye due to gray cataract was treated every three days with 0.4 ml, 1 Casein-Unit, of Multi-Protein preparation. After 5 treatments the study had to be terminated due to other medical reasons but an obvious reduction in opacity was observed and the lady reported improved vision on the affected eye. No irritation, pain or other discomfort could be observed during the treatment or 1 month post-treatment.

A suitable dose range for this indication is 0.01 to 50, preferably 0.1 to 5 mg per treatment.

### Clinical Examples - Pain

Pain is initially caused by the mechanical rupture of tissues and the acute pain sensation is fading away shortly after the trauma. A certain tenderness remains throughout the early phases of the healing process. Infections and acute inflammations may cause severe pain due to swelling of tissues, chemical reactions, biochemical activities, forming of necrotic tissue, adhesions and scar formations. The pain may remain for a long time after terminated healing or even be permanent.

Pain is a very subjective parameter and it is extremely hard to interrupt clinically. By definition, pain is everything from light itching to severe disabling pain. Patients suffering from severe pain have a tendency of growing accustomed to the pain and their acceptance level is continuously elevated.

The onset of pain relief was the quickest and most obvious effect of the test preparations according to the invention. Pain relief was reported 20 minutes to 2 hours after the first application and in most cases the pain was reduced to a mild level or only a tenderness after 2 days. Independent of indication, acute or chronic pain, the patients reported an identical pattern of pain relief. Sense of feeling and touch did not disappear from the treated areas.

Patients that did not experience any pain before treatment, reported a smoothing and alleviating feeling after the first treatment, especially in treatments of the eye and mouth-cavity. (Cf. the Examples above, pain is a parameter in most of these studies.)

In urinary bladder infections and gum infections the pain sensations led to physico-social disturbances of the patients. With the quick on-set of pain relief from test preparation the patients regained their ability of concentration and were able to act as normal people within 2 days of treatment. (Compare Examples 3 and 10.)

The most objective scoring of pain sensation was performed in inflamed horse joints where the pain sensation was related to the time for supporting an affected leg. The results are comparable to the general results of studies in humans (compare Example 11).

A suitable dose range for this indication is 0.01 to 200, preferably 1 to 25 mg per treatment.

### Clinical Examples 20-40 - Various indications

### Example 20 - Gastric ulcer

A man of 40 having recurring complaints characteristic of a mild form of gastric ulcer was treated with acid-resistant gelatin capsules containing 5 mg PHIM per capsuie. He swallowed 1 capsule a day with a glass of water for 2 weeks. After about 4 days the stomach complaints disappeared and the stomach worked quite perfectly with natural faeces and no pain etc.

A 55 years old man having gastric ulcer complaints constantly recurring for 20 years was treated with the same dosage as above. Already after a few day's treatment the complaints had disappeared and his stomach worked normally.

Gastric ulcer is an inflammatory process probably starting due to a bacterial attack First the intestinal mucosa is infected by the microbe and then the condition transforms to an inflammation which soon is converted to an ulcer. This means that there is a situation where the cells of the intestinal mucosa become divergent. The situation is quite analogous to that of skin infections and wounds; the difference between the skin and the intestinal mucosa is the presence in the intestinal mucosa of a cell system named Peyer's patch cell system consisting of highly active B-cells, T-cells, etc. This system is regarded to be the gateway to our autoimmune diseases. Owing to the fact that PHIM works in this cell system and has the ability to remove divergent cells from the sensitive mucosa layer, it should work, quite logically, also on autoimmune diseases like Crohn's disease, ulcerative colitis, rheumatoid arthritis, etc.

A suitable dose range for this indication is 0.5 to 300, preferably 1 to 50 mg per treatment.

### Example 21 - Treatment of wrinkled skin

Wrinkled skin to a major part is caused by free radicals crosslinking the collagen. The formation of free radicals is caused by warned-out and dead cells being an excellent growth medium for bacteria speeding up in turn the formation of free radicals. By allowing PHIM to "eat" the dead cells and the bacteria. the cause of wrinkles is removed.

A 33 years old woman was treated each night for 60 days with a gauze bandage moistened with a PHIM 106 solution. The total amount of PHIM 106 used each night was about 0.15 mg. The bandage was allowed to be in close contact with the skin for 30 minutes.

By treating only the area below one of the eyes, the other side was used as a control. Already after 15 day's treatment one could observe a difference in the elasticity of the skin and after totally 60 day's treatment there was a visually apparent difference as regards wrinkles. The treated skin area was very soft and elastic and the number and depth of the wrinkles had decreased considerably compared to the untreated skin area.

A suitable dose range for this indication is 0.01 to 100, preferably 1 to 25 mg per 100 cm².

### Example 22 - Polyps

A man of 62 having a polyp in his anus was treated. The man had suffered from the polyp for 3 years and had been treated by a physician. He had been treated with different kinds of medicines but no improvement was observed.

He was treated with a gauze bandage containing about 5 mg of PHIM dissolved in 5 ml of saline. The treatment was repeated totally 5 times. All trouble disappeared and at the next visit to the physician it was observed that the polyp had disappeared. 8 months after the treatment, the man is still free of complaints.
A suitable dose range for this indication is 0.5 to 250, preferably 1 to 50 mg per treatment.

### Example 23 - Warts

A 35 years old man having a wart on his neck was treated for one week with a plaster ("Hansaplast") containing a solution of PHIM 106, about 0.1 mg/plaster. The treatment was repeated each day during a week. After 1 week's treatment the wart had completely disppeared.

A suitable dose range for this indication is 0.5 to 250, preferably 1 to 50 mg per treatment.

### Example 24 - Common cold

5 patients in the age of from 30 to 63 years were treated with PHIM 106 only a few hours after the outbreak of the first cold symptoms. The treatment was carried out with nasal sprays every 4 hour and with mouth washes every 6 hour. The dose used in the nasal spray treatment was about 0.1 mg in each nostril at a time and in the mouth washing treatment about 1.5 mg/wash. When washing the mouth the solution was kept in the mouth for about 2-4 minutes whereupon the solution was swallowed. After 12 hours the common cold symptoms had disappeared and the patients were free of complaints.

A suitable dose range for this indication is 0.1 to 100, preferably 1 to 25 mg per treatment.

### Example 25 - Haemophilus influenza

A woman of 34 had recurring sinusitis by infection of Haemophilus influenza. A few hours after the appearance of the symptoms of pressure and pain in the nasal sinus, the mouth was washed for 3 minutes with a solution of 2 mg of PHIM 106 every two hours totally 4 times and about 0.1 mg of PHIM 106 was sprayed into each nostril. The spray treatment was repeated each three hours for a total of 3 days. The pressure caused by the nasal sinus infection disappeared already a few hours after the first treatment and the secretion from the nose strongly increased. After 3 day's total treatment the woman was free of complaints.

A suitable dose range for this indication is 0.1 to 100, preferably 1 to 25 mg per treatment.

### Example 26 - Herpes Zoster

A 70 years old man having a very painful Herpes Zoster infection in his face since 10 months back was treated topically with a gauze bandage containing about 1-2 mg of PHIM, every three days.

Already after the first treatment the itch was reduced and also the pain, to disappear completely after 12 days. Owing to the infection, the man had had difficulties in chewing owing to pain in the palate, but after 12 days he was able to chew the food with no problems.

A suitable dose range for this indication is 0.01 to 100, preferably 1 to 25 mg per 100 cm².

### Example 27 - Herpes genitalis

A man of 62 having Herpes genitalis since 10 years back was treated. The complaints recurred regularly every 4 month and during the time of acute complaints the man abstained from sexual intercourse.

He was treated with a bandage soaked with a PHIM 106 solution, about 3 mg/bandage. The treatment was repeated twice a day for 2 days. His complaints disappeared already after the second treatment. Since the completion of the treatment, the man has had no complaints during the last 10 months.

A suitable dose range for this indication is 0.01 to 100, preferably 1 to 25 mg per treatment.

### Example 28 - Hemorrhoids

Two persons were treated for hemorrhoids, one man of 55 years and one woman of 30 years. The woman had had complaints for about 3 years with pain and minor bleedings. She was treated with dry PHIM 106 powder wrapped in gauze bandage which was applied onto the area concerned. Each dose was about 5 mg. 5 treatments in total were needed before disappearence of the complaints. She has been totally free of complaints for one year now.

The man had been troubled off and on during the last years. Six months ago the complaints became acute with bleedings and very strong pain as a result thereof. The man was treated once with a gauze bandage soaked with about 4 mg of PHIM 106. The pain disappeared within about 20 minutes and after this single treatment all complaints disappeared and have not recurred since then.

A suitable dose range for this indication is 0.5 to 250, preferably 1 to 50 mg per treatment.

### Example 29 - Tourist diarrhoea

A 41 years old man acutely developed food poisining (probably from Staphylococcus) with diarrhoea and vomits. One hour after the man had fallen ill he was tretaed with 5 mg of PHIM 106 by keeping it in the mouth for about 3 minutes whereupon it was slowly swallowed. This procedure was repeated 3 times every second hour. After the fourth treatment the stomach pains had disappeared and the vomits and the severe diarrhoea totally ended.

A suitable dose range for this indication is 0.5 to 300, preferably 1 to 50 mg per treatment.

### Example 30 - Thin-hairness

2 men were treated, 55 years old and 62 years old, respectively. They both had suffered from thin-hairness for the last 10 years. The treatment was carried out by soaking the entire scalp with a solution of PHIM 106, totally about 5 mg PHIM per treatment. In order to maintain the humidity in the scalp, it was covered with a shower cap for 30 minutes. The treatment was repeated once a week for about 3 months. After this time of treatment fresh hair began to grow out.

The reason for the good result seems to be that PHIM 106 effectively decomposes all dead and divergent cells being a good nutrient substrate for bacteria. Said bacteria produce toxins which locally trigger the cells of the skin layer to produce i.a. TNF, tumour necrosis factor, which in turn, when present in large amounts, affects the hair growth negatively. By removing said dead and divergent cells including bacteria also the microcirculation is affected in favour of a renewal of the hair.

A suitable dose range for this indication is 0.01 to 100, preferably 1 to 25 mg per 100 cm².

### Example 31 - Acne

2 women, age 29 and 30 years, were treated for acne in the face. The woman of 29 had severe complaints whereas the 30 years old woman had moderate complaints, mainly in the forehead.

They were treated with about 0.1 mg PHIM 106 several times a day for 4-6 days. The effect was quite remarkable already after the first treatment and after a few days of treatment the very infection had disappeared and the cure was almost total. One week after the treatment only pigment traces were evident.

A suitable dose range for this indication is 0.01 to 50, preferably 1 to 10 mg per treatment.

### Example 32 - Bronchitis

A 55 years old man had relatively bad bronchitis complaints. The complaints manifested themselves in the form of respiration complaints, difficulty to walk longer distances than 100 meters without a break and severe tiredness as well as annoying hacking cough.

The cause of the bronchitis was considered by the physician to be a Mycoplasma infection which occurred 3 years earlier and which in spite of antibiotic treatment developed resistance. The infection also led to the formation of water in the pleura which was verified by X-ray examination.

The patient was treated with mouth washes, about 4 mg PHIM 106 each time. The PHIM solution was kept in the mouth for about 4 minutes and then it was slowly swallowed. This treatment was repeated during the first two weeks every second day. During this time also small amounts of PHIM 106 were inhalated.

During the first two weeks no improvement was observed but after two weeks' treatment the lymph gland on the left side of the neck swelled resulting in pain. During this time the treatment was continued about 3 times. After about 1.5 weeks all problems regarding the lymph nodes had disappeared and the bronchitis complaints of the patient began to subside. After further 3 weeks' treatment with mouth washes every fourth day the bronchitis complaint had completely disappeared.

After this total treatment extending over 6.5 weeks the patient had recovered completely and after a short time he could walk 5 kilometers with no problems.

A suitable dose range for this indication is 0.1 to 100, preferably 1 to 25 mg per treatment.

### Example 33 - Prostatitis

A man of 52 years suffered from prostatitis complaints each winter since the age of 20. During the last 4 years said complaints became more acute resulting in extremely severe abdominal pain. In every acute phase he was treated with different kinds of antibiotics but as soon as the antibiotic treatment was completed, the complaints recurred within some week. At such a recurring prostatitis problem of mild type, the man received acid-resistant capsules containing PHIM 106; about 5 mg/capsule. He took 2 capsules/day for one week totally. All symptoms disappeared and the man has had no single recurrence during the last 12 months.

A suitable dose range for this indication is 0.5 to 300, preferably 1 to 50 mg per treatment.

### Example 34 - Resistant Strain of Mycoplasma Infection

A 55 years old man having a resistant strain of Mycoplasma was treated. The man fell ill acutely 3 years ago and was treated with different kinds of antibiotics, but owing to underdosing a resistant form developed. Some weeks after the infection the man contracted high fever with a very severe cough as a result and in X-ray examination water in the pleura was observed.

The complaints of the man manifested themselves in the form of respiration complaints, difficulty to walk longer distances than 100 meter without a break and severe tiredness as well as annoying hacking cough.

The patient was treated with mouth washes, about 4 mg PHIM 106 each time. The PHIM solution was kept in the mouth for about 4 minutes and then it was slowly swallowed. This treatment was repeated during the first two weeks every second day. During this time also small amounts of PHIM 106 were inhalated.

During the first two weeks no improvement was observed but after two weeks' treatment the lymph gland on the left side of the neck swelled resulting in pain. During this time the treatment was continued about 3 times. After about 1.5 weeks all problems regarding the lymph nodes had disappeared and the bronchitis complaints of the patient began to subside. After further 3 weeks' treatment with mouth washes every fourth day the bronchitis complaint had completely disappeared.

After this total treatment extending over 6.5 weeks the patient had recovered completely and after a short time he could walk 5 kilometers with no problems.

A suitable dose range for this indication is 0.1 to 100, preferably 1 to 25 mg per treatment.

### Example 35 - Mastitis in human

A woman, 28 years old, got very severe galactostasis complaints already 3 days after delivery manifesting themselves in lactiferous glands hard as stone and an intense pain. These complaints set in already half an hour after breast-feeding. She was treated at the Academic Hospital in Uppsala, Sweden, with various methods available, but nothing helped.

A solution of about 0.1 mg PHIM 106 was dropped onto the nipple of the woman when the intense pain began. All pains disappeared within about 15 minutes and after about 45 minutes the hard lactiferous glands had become soft.

This treatment was repeated after each breast-feeding for more than 3 months. In order to check whether the treatment was needed all the time, the woman sometimes refrained from treatment directly after breast-feeding before onset of the pain. At each occasion this check was made the intense pains and the hard lactiferous glands recurred.

A suitable dose range for this indication is 0.01 to 25, preferably 0.1 to 5 mg per treatment.

### Example 36 - Allergic Itch

A woman, age 28, got allergic problems in the form of intense itch with nettle rashis above one of the knees and also on and below the chin. The rash looked like white, somewhat spread elevations on the knee whereas the chin was totally spotted with small rash of the same colour as the skin.

A gauze bandage was wetted with PHIM 106 solution, about 2 mg. The gauze was attached above the rash on the area above the knee. After 10 minutes the gauze bandage was removed. 45 minutes later the itch began to fade out and had completely disappeared after 1.5 hours. The white elevations had also disappeared and the nettle rash had faded.

24 hours later the area under and on the chin was treated. This area now itched intensively and was more irritated than before owing to the woman's scratching.

Directly upon application of the gauze bandage with the PHIM solution, the itch increased and the gauze bandage was removed after 7 minutes owing to a very intense itch. During a period of 1.5 hours the itch declined and completely disappeared 1.45 hours later.

No problems whatsoever were observed neither on the area at the knee nor in the face after 48 and 24 hours, respectively.

A suitable dose range for this indication is 0.01 to 100, preferably 1-25 mg per 100 cm².

### Example 37 - Anti-adhesion of tendon to sheath

The purpose of the study was to investigate the anti-adhesion properties of Multi-Protein from Krill in healing of ruptured Achilles tendon in rabbits. The left Achilles tendon of two rabbits was ruptured and immediately sutured in a split/overlapping technique enabling a non-immobilized left leg post-op.

On Day 5 post-op. there was an evident adhesion between the tendon and the sheath and 1 mg (0.25 ml) of the Multi-Protein was injected interstitially in the sheath on Day 5 and repeated on Day 7 and 9 post-op. 24 hours after the first injection there was no evident adhesion between tendon and sheath and again the tendon glided freely in the sheath. Six weeks post-op. the adhesion had not recurred.

Eight months post-op. the animals were sacrificed and the tendons were macroscopically inspected for adhesions, surplus of fibrin and collagen. The tendons and sheaths had healed separately and no signs of adhesions could be observed. The tensile strength of the operated left leg tendons were compared to the unoperated right leg tendons and no difference in tensile strength could be detected.

Multi-Protein specifically decomposes the surplus of fibrin without affecting the fibrin need for a proper healing of tendon and sheath, respectively.

A suitable dose range for this indication is 0.1 to 100, preferably 1 to 10 mg per treatment.

### Example 38 - Detachment of adhered wrist

The purpose of the study was to investigate the efficacy of Multi-Protein in the decomposition of fibrinous tissue in wrists with reduced mobility.

A woman in her 40's suffered from a stiff wrist due to a long immobilisation of the joint with hard plaster after a complicated fracture of the arm, 25% of normal mobility.

Despite a training programme the mobility of the affected joint improved poorly and the diagnos was fixation due to fibrin coating inside the joint.

Multi-Protein was injected, 2 mg (0.5 ml), intraarticularily for totally 4 times, 3 days apart. The mobility improved successively over 14 days to 50-60% mobility. This woman recovered a mobility to 70-80% after 4 months on training.

No adverse reactions were observed.

A suitable dose range for this indication is 0.1 to 100, preferably 1 to 10 mg per treatment.

### Example 39 - Anti-thrombolytic/anti-embolic properties of Single-Protein

The purpose of the study was to investigate the efficacy of Single-Protein from Krill in the treatment of thrombi and emboli.

The thrombi were caused by an artifical stasis of the main ear vein till a proper thrombus had developed. Single-Protein was injected, 0.5 mg (0.2 ml), into vein in the direction towards the thrombus, 2 cm from the ischemic area. Within 30 minutes the thrombus was completely dissolved and the blood had free passage. Small necroses developed in the area but these were resorbed within 7 days.

In the control animals the ischemic area turned totally necrotic within 4-5 days.

A suitable dose range for this indication is 0.1 to 200, preferably 1 to 10 mg per treatment.

### Example 40 - Treatment of Glaucoma

The purpose of the study was to investigate the efficacy of Multi-Protein in the treatment of increased intraocular pressure.

A man, age 74, experienced a slight pain/disconfort in the right eye and it was found that he suffered from an increased intraocular pressure. At check-ups it was established that the increase of pressure was permanent and not caused by acute reasons.

Every four day 0.1 mg of Multi-Protein was dropped into the affected eye for totally three times. Two weeks post-treatment the intraocular pressure was normal and still 4 months post-treatment the pressure was normal.

The man experienced an immediate pain relief, within 20 minuts, at the first treatment.

No adverse reactions were observed.

A suitable dose range for this indication is 0.01 to 50, preferably 0.1 to 5 mg per treatment.

### Example 41 - Anti-viral effect on HIV-contaminated cell lines, in vitro

This study was carried out at the Swedish National Bacterological Laboratory (SBL). Multi-Protein was compared to AZT and Foscarnet. All preparations were tested at dilutions from undiluted to 10⁻⁵ (highest concentration for Multi-Protein was 5 mg/ml). The preparations were added to the cell linjes, HIV-I infected to 40-60%, under identical and standardized conditions.

At low dilutions of AZT and Foscarnet, all cells in the cultures were attacked and killed. Only dead and collapsed cells were detected in these cultures.

Multi-Protein, at low dilutions, shows inhibiting effect on the virus. In the cultures 40-60%, identical to the initial values in resp. culture, of the cells were attacked and they were detached from surface. These attacked cells showed morphological changes and the virus was unable to proliferate. The uninfected cell in the Multi-Protein group were not attacked, neither by the Multi-Protein nor by liberated viruses from attacked cells; they stuck to the surface and showed no signs of morphological changes.

Thus, it is shown that Multi-Protein from Krill may distinguish between healthy and divergent cells, such as viral host cells, and may specifically recognize, entrap and destroy only the divergent matters without causing harm to the healthy cells.

A suitable dose range for this indication is 1 to 500, preferably 10 to 300 mg per treatment.

| **Inhibiting efficacy - Trial 1** | | |
|---|---|---|
| | Virus (IC50) | Cell viability (tox.) |
| | (HIV-1, strain HTLV IIIB moi 0.4) | |
| Multi-Protein | 200 | 100 (deformed cells) |
| AZT | 100 | 100 |
| Foscarnet | 200 | 100 |

Control: 50% infected cells. Cell toxicity and percentage of infected cells measured by immunofluorescence.

| **Inhibiting efficacy - Trial 2** | | |
|---|---|---|
| | Virus (IC50) | Cell viability (tox.) |
| | (HIV-1, strain HTLV IIIB moi 0.2) | |
| Multi-Protein | 200-400 | 100-200 (deformed cells) |
| AZT | 100 | 100 |
| Foscarnet | 100 | 100 |

Control: 50% infected cells. Cell toxicity and percentage of infected cells measured by immunofluorescence.

### Example 42 - Athlet's foot (Epidermophytosis)

The purpose was to study the effectiveness and usefulness of Multi-Protein in epidermophytosis of the foot.

41 patients with fungous infections were included in this study, treated once a day with a Multi-Protein foot bath, 5 cu/ml, for 30 minutes for 3 days and with Hydrogel, 5 cu/ml, overnight, for maximum 7 days.

The pain relief was instant in many patients and totally gone within 2 days for others. Plaques over open surfaces, in cracks and under nails were easily removed and all signs of plaque, smell and infections were gone after three days.

A suitable dose range for this indication is 0.01 to 100, preferably 1 to 25 mg per 100 cm².

### Example 43 - Eczema infections

The purpose was to study the effectiveness and usefulness of Multi-Protein in eczematous seborrheic and psoriasis infections.

Fourty patients were treated once to twice daily with Multi-Protein Hydrogel, 2.5 cu/ml.

Patients with dry eczema/plaque showed no signs of inflammation or infection after 2-4 treatments, 1-2 days. The fatty type of seborrheic plaques disappeared after 6-9 days, though the inflammations/infections had vanished within the initial 2-4 days.

Patients with psoriasis piaque experienced an improved efficacy from their normal steroid creams, probably due to the effective removal of the plaque by Multi-Protein which resulted in better access of the steroids into the skin.

A suitable dose range for this indication is 0.01 to 100, preferably 1 to 25 mg per 100 cm².

### Example 44 - Prepuce infection

The purpose was to study the effectiveness and usefulness of Multi-protein in prepuce infections in infants.

Two infants, 4 resp. 6 weeks old, were treated twice daily with 1 cu/ml, Multi-Protein solution. Approx. 10 ml of the solution was flushed under the prepuce morning and evening, using a standard syringe with a soft catheter. After 3 days both the infants were free from symptoms and the infections did not recur within a 2 months' follow-up.

A suitable dose range for this indication is 0.01 to 100, preferably to 1 to 25 mg per treatment.

### Example 45 - Prepuce infections in dogs

The purpose was to study the effectiveness and usefulness of Multi-Protein in prepuce infections in dogs.

Six dogs were flushed under the prepuce once daily with the Multi-protein solution, 1 cu/ml. 10 ml of the Multi-Protein solution were sucked into a standard disposable syringe. A soft silicone catheter was attached to the syringe and the catheter was inserted under the prepuce and the area was slowly flushed. Approx. 1 ml of the solution was kept under the prepuce for minimum 2 minutes and the dogs were kept from licking the area for 30 minutes.

The purulent exudation stopped within 2 days in all the cases and all signs of infection and inflammation were gone within 4 days.

A suitable dose range for this indication is 0.1 to 100, preferably 1 to 25 mg per treatment.

### Example 46 - General purpose eye drops

The purpose was to study the effectiveness and usefulness of Multi-Protein in "tired and irritated" eyes but without any signs of clinical inflammation or infection.

Twelve patients with no record of eye diseases, allergic reactions to air pollutants or eye infections/inflammation were treated ad hoc for "tired and irritated eyes". 2 drops of Multi-protein solution, 0.1 cu/ml, were dropped into the eyes whenever needed.

All patients experienced an immediate alleviation of tension/weak pain, within 30 minutes. No one reported any change in light sensitivity, focusing or any dilatation of the pupils. Sometimes a transitory dryness was experienced.

No irritation, increase in lacrimal secretion or other adverse reaction was observed.

A suitable dose range for this indication is 0.01 to 50, preferably 0.1 to 5 mg per treatment.

### Example 47 - Abscesses in calves

The purpose was to study the effectiveness and usefulness of Multi-protein in abscesses in calves, using Multi-Protein solution, 5 cu/ml.

Two calves with abscesses, approx. size of 25 to 40 ml, on the neck, formed after injection with Calcium solution, were treated once daily. 10 ml of the Multi-Protein solution were instillated through a drainage tube and kept in place for min. 4 hours. Then the tube was openend and left so till the next treatment.

After the third treatment the drainage fluid was clear and after the sixth treatment the tubes were removed. The "pockets" healed completely after 9 resp. 12 days.

A suitable dose range for this indication is 0.1 to 200, preferably 1 to 20 mg per treatment.

### Example 48 - Boils in dogs

The purpose was to study the effectiveness and usefulness of Multi-Protein in infected boils on the paws of dogs.

Three boxers with painful, infected and excudating/bleeding boils between the toes were treated twice daily with Multi-Protein solutions, 5 cu/ml.

A gauze was drained with 2 ml of the solution and then applied over the boil. The paw was bandaged to keep the gauze fixed and a rubber boot was used for protection.

An obvious pain relief could be observed after 1 day and the purulent exudation/bleeding stopped within 2 days. After 5.7 days' treatment only slight signs of inflammation could be observed and after 11-15 days all signs of the boils were gone and the dogs could move around freely.

A suitable dose range for this indication is 0.1 to 100, preferably 1 to 25 mg per treatment.

### Doses for various indications

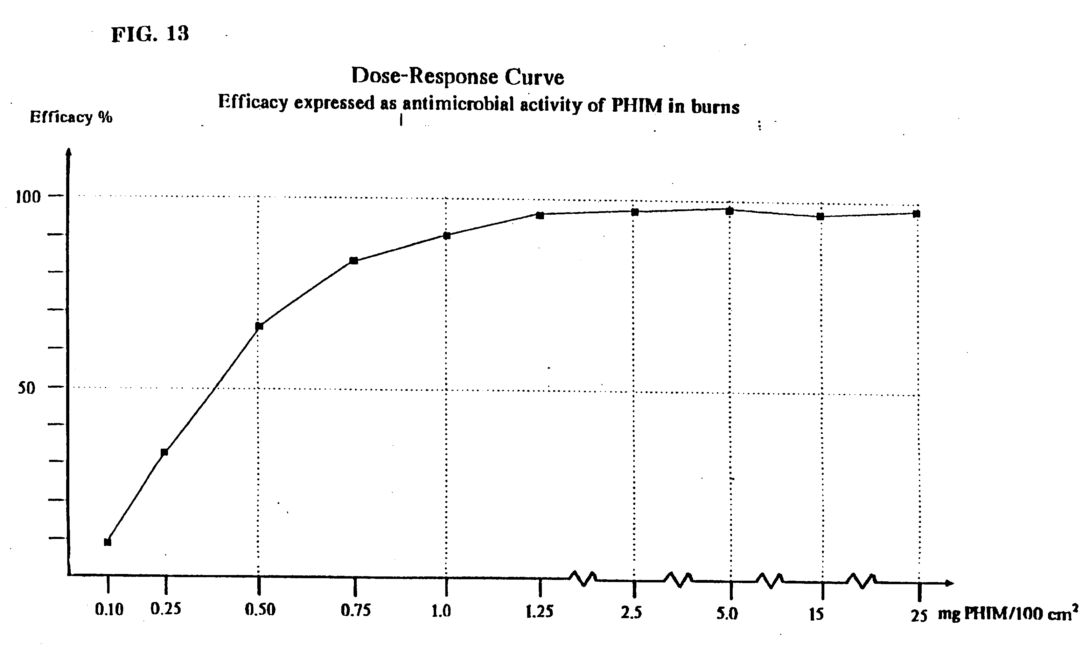

**Figure 13** shows the Dose-Response curve for one specific indication, namely burns, and shows the percentage efficacy, expressed as antimicrobial activity, as a function of the amount of PHIM in mg per 100 cm² of wound area. An almost 100% efficacy is reached at a dose of about 2 mg of PHIM per 100 cm² wound area. The Dose-Response curve shown would be representative also for other applications.

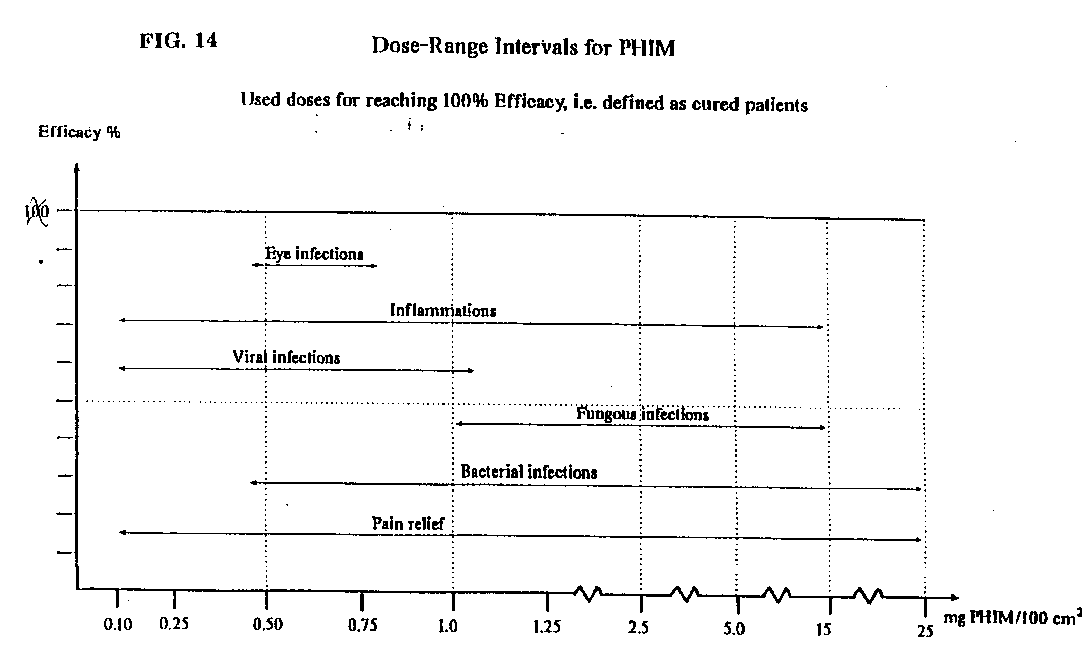

**Figure 14** shows Dose-Range intervals for PHIM, i.e. used doses for reaching 100% efficacy, defined as cured patients, for various indications. The efficacy is plotted against the amount of PHIM in mg per 100 cm² of afflicted area. The approximate dose ranges are (mg PHIM/100 cm² afflicted area):
- eye infections 0.5-0.8
- inflammations 0.1-15
- viral infections 0.1-1
- fungous infections 1-15
- bacterial infections 0.5-25
- pain relief 0.1-25

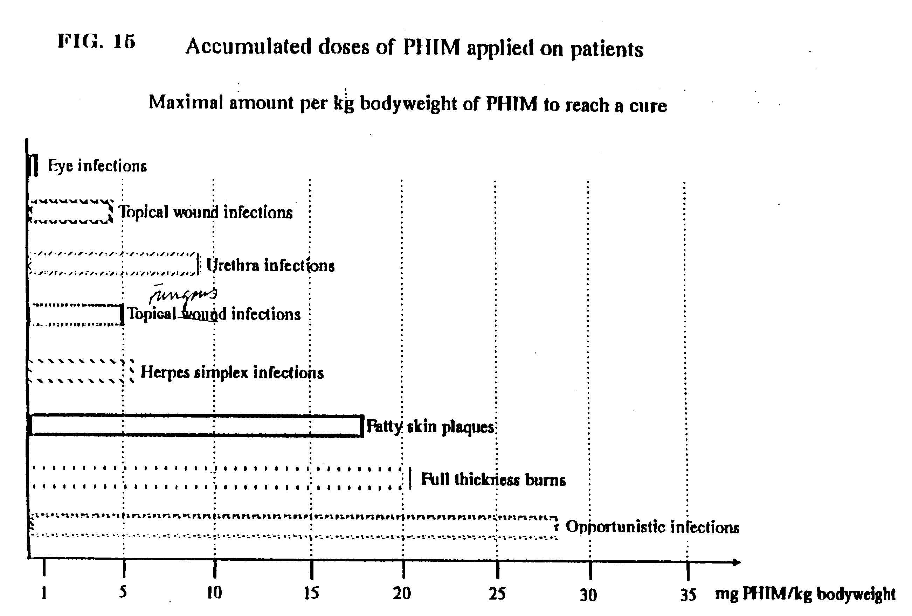

**Figure 15** shows the maximal accumulated amount per kg body weight of PHIM to reach a cure, i.e. the clinical end-point, for various indications. The approximate maximal amounts for various indications are (mg PHIM/kg body weight):
- eye infections 1
- topical wound infections 5
- urethra infections 9
- fungus infections 5
- Herpes simplex infections 5
- fatty skin plaques 18
- full thickness burns 20
- opportunistic infections 28

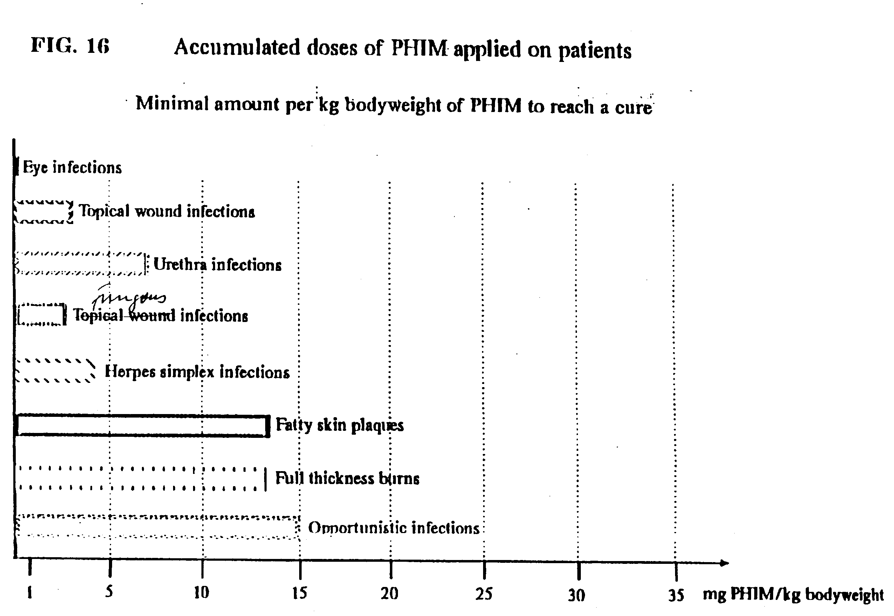

**Figure 16** shows the minimal accumulated amount per kg body weight of PHIM to reach a cure for various indications. The approximate minimal amounts for various indications are (mg PHIM/kg body weight):
- eye infections <1
- topical wound infections 3
- urethra infections 7
- fungus infections 3
- Herpes simplex infections 4
- fatty skin plaques 13
- full thickness burns 13
- opportunistic infections 15

### Conclusions of the Clinical Examples 1 to 51

Single-Protein and Multi-Protein preparations according to the invention present a total range of actions that seem to resemble the mode of action of phagocytizing cells.

No clinical limitations or drawbacks could be established from the investigated indications. On the contrary, the preparations exhibit good efficacy in clinical situations where the natural immune defence was not responding, e.g. wounds in stasis, and where the immune defence was completely suppressed, e.g. opportunistic infections.

### Adverse Reactions

No adverse reactions were observed in any of the many patients included in the above reported studies. Patients suffering from poly-allergies, sead food allergies, sensitive skin, dry eyes and patients with known suppressed resp. hyperactive immune system have been included in studies or tested separately without exhibiting any signs of adverse reactions.

It has been shown that the claimed Multi-Protein and Single-Protein preparations are atoxic.

Based on these full and preliminary clinical investigations, it can also reasonably be concluded that the inventive substanses and compositions would be effective as a cure of a very great number of other diseases and conditions, where the immune system is responding, such as glaucoma; trachoma; cancer; AIDS/HIV; autoimmune disease such as Rheumatoid arthritis, Grohn's disease, multiple sclerosis, ulcerative colitis; diseases such as cholera, leprocy, malaria, hepatitis, typhoid fever; sepsis; transplantation of organs and grafts (prophylaxis), techniques; cosmetics.

### Clinical Example 49 - Cancer

This study was carried out by Prof. Takashi Makita, Yamaguchi University, Japan.

### Background

Cancer diseases are very complex and consist of many different diseases. The complexity is increasing when the tumours are growing and the primary tumour is spreading metastasis to other organs as well'as into the lymphnodes. Additional to the increasing burden of the tumours are the opportunistic infections, which occur partly as a result of a depressed immune defence system, caused by the tumour itself, but also as a result of the conventional treatment with chemotherapy, which is the most common treatment used today for treatment of cancer.

Many different problems are connected with the use of traditional chemotherapy. The major problem is that anticancer drugs do not have the properties to distinguish between healthy tissue cells vs cancer cells. This is the most serious problem, because the white blood cells needed for the immune defence are destroyed by the chemotherapy treatment. Consequently, it is not possible to treat cancer patients in an optimal way.

The depressed immune defence results in increased tumour burden as well as uncontrollable opportunistic infections. The combination of limited possibilities for optical treatment and opportunistic infections, decrease the quality of life of the patients who are suffering from cancer diseases. There are very few well controlled studies in humans and in general the efficacy is normally around 20-25%, depending on the type of cancer.

In a well controlled phase II study performed in Japan in patients suffering from primary and secondary liver cancer; the response rate (CR and PR) was 9.5% for Adriamycin and 20% for Mitomycin C. See T. Taguchi et al, Regional Cancer Treatment (1992) 4:161-165.

In animal studies the efficacy is normally higher depending on the fact that higher doses of anticancer drugs are given compared with treatment of human patients, because adverse reactions are very difficult to study in animal models, with the exception of objective parameters. It is also very difficult to compare historical data.

However, Cisplatin has been used in rats with implanted Yoshida Sarcoma and tumour cells have been removed from the tumour and cultured with the purpose to study surviving cells, the result being that 53% of the cells are not killed by Cisplatin. See K.R. Harp et al, Antitumor, Toxic and Biochemical Properties of Cisplatin.

Similar experiments have been performed with Mitomycin C in doses up to 0.5 mg/kg. In that case the efficacy for non-resistant Yoshida Sarcoma was almost 100%, calculated as 30 days of survival of the rats. The dose of 0.5 mg/kg used in rats is impossible to use in human because of severe adverse reaction such as bone morrow depression, which is lethal for the patients. Yoshida Sarcoma easily becomes resistant to Mitomycin C and in that case no effects could be detected despite the high dose of 0.5 mg/kg or even higher doses.

The side effect profile with the mentioned dose level as well as lower doses includes loss ofbody weight, hair loss, decreased leucocytes and chronic diarrhoea. See Mitomycin C, Kyowo Hakko Ltd., Tokyo, Japan. Anticancer agents as Adriamycin, Cisplatin, Mitomycin C etc., are very toxic and can be used only for intra-venous and intra-arterial administration.

Furthermore, the tumour cells very often develop resistance to the anti-cancer agents in a similar way as bacteria do develop resistance to antibiotics. The development of resistance is a severe drawback for curable treatment.

### Summary of test procedure and results

The purpose of the study was to investigate the efficacy and usefulness of PHIM 106 on treatment of Yoshida Sarcoma in rats.

PHIM 106 was injected in three different routes, intraperitoneally (i.p.), intratumorally (i.t.) and subcutaneously (s.c.) to white Wistar rats with implanted Yoshida Sarcoma. PHIM 106 was used as a single injection of 5 mg/kg i.p., i.t. and s.c. and as repeated doses of 1.25 mg/kg s.c. twice a day during 7 days, 5 mg/kg s.c. every second day for a total of 4 injections, and 12.5 mg/kg s.c. every second day for a total of 4 injections.

The treatment was started when the size of the implanted tumour was 10 mm x 10 mm.

The rats were sacrificed 7 days after finished injection. The size of the tumours was measured and compared with the untreated control rats.

The reduction of the size of the tumour was 46% in rats treated i.t. with a single dose, 56% in rats treated s.c. with a single dose and 49% in rats treated i.p.

In the group treated with repeated doses of 1.25 mg/kg twice a day during 7 days s.c. the efficacy was 72%.

In the groups receiving 5 mg/kg and 12.5 mg/kg s.c. every second day for a total of 4 injections, the tumour responses were 53% and 69% respectively.

One rat in the group receiving 12.5 mg/kg had negative tumour growth. The degree of metastasis in the treatment groups was very small compared to the control rats.

The treated rats showed normal behavior regarding drinking and eating, in contrast to the control rats. No adverse reactions could be observed during this trial.

### Description of PHIM 106

As explained above, PHIM 106 is an abbreviation for proteins having enzymatic properties and originating from marine sources, in the below tests from antarctic krill. According to the invention fundamental new properties have been discovered of these proteins. The hypothesis is that the proteins first recognize sick and divergent cells as well as microbes and then destroy the target cells by the enzymatic properties. Nothing happens with the healthy tissue cells.

The mechanism behind the sharp selectivity is under investigation, but the working hypothesis is that the proteins are capable of reacting on the same signals as our own immune defence system, and attack all foreign invaders as well as divergent cells.

Cancer cells are divergent in the sense that they express fragments of tumour proteins on the cell surface and they will be recognized as divergent by the immune defence system compared with healthy tissue cells.

PHIM 106 has been used in open human studies in more than 400 patients without any kind of side effects.

In animal no toxicity can be detected. Up to 5 g of PHIM 106/kg body weight of rats has been injected i.v. without any sign of toxicity.

As PHIM 106 consists of proteins with a size of 24-34,000 Daltons and origin from marine sources formation of antibodies would be expected, but no formation of antibodies could be detected when rabbits and guinea pigs were immunized.

### Materials and methods

### Aim of study

To investigate in small comparative studies the routes of administration, the sequence between the injections and a dose response curve and an overall usefulness of PHIM 106 using white Wistar rats with implanted Yoshida Sarcoma.

A second objective was also to study the targeting properties of PHIM 106 by using it for treatment of the tumour from the veins as well as from the lymphatic vessels.

### Design of the study

Comparative study with injection of PHIM 106 as a single injection i.p., i.t., s.c., repeated doses every two day and a total of 4 injections s.c. The control groups was untreated. The treatment was started when the implanted tumour cells had a size of about 10 mm x 10 mm.

The rats were sacrificed 7 days after the treatment was finished and the size of the tumour were measured. Histopathological examination was performed.

### Formulation of PHIM 106

Lyophilized white powder without preservatives or antimicrobial additives.

The white powder was reconstituted in Saline for injection to a final concentration of 5 mg/ml solution.

### Procedure

1 x 10⁴ Yoshida Sarcoma cells were implanted subcutaneously on the back of white Wistar rats. The treatment of the rats started when the size of the tumours was 10 mm x 10 mm.

PHIM 106 was injected in three different routes, intraperitoneal (i.p.), intratumoral (i.t.) and subcutaneously (s.c.) about 3-5 cm from the tumour in the healthy part of the skin.

The animals were divided into the following 7 groups.
- Group 1.: The control group of 11 rats without treatment.
- Group 2.: 5 rats treated with a single injection of 5 mg PHIM 106/kg directly into the center of the tumour (i.t.).
- Group 3.: Treated with a single injection of 5 mg PHIM 106/kg subcutaneously (s.c.).
- Group 4.: 1 rat treated intraperitoneally (i.p.) with a single injection of 5 mg PHIM 106/kg.
- Group 5.: 3 rats treated subcutaneously (s.c.) with 1.25 mg PHIM 106/kg twice a day during 7 days.
- Group 6.: 5 rats treated subcutaneously (s.c.) with 5 mg PHIM 106/kg every second day for a total of 4 injections.
- Group 7.: 5 rats treated subcutaneously (s.c.) with 12.5 mg PHIM 106/kg every second day for a total of 4 injections.

Seven days after the treatment was finished, the rats were sacrificed and the volume of the tumors was measured. The rats in the control group were sacrificed at the same day as in the treatment groups.

Histopathology was also performed of the tumour in all the groups.

Blood was collected and stored for later examination.

### Results

In group 2, treated with a single injection i.t. of 5 mg PHIM 106/kg, the reduction of the tumour compared with the control was 46%.

In group 3, also treated with single injection s.c. of 5 mg PHIM 106/kg, the reduction of the tumour was 56%, and in group 4, treated as 2 and 3 but i.p. the reduction of the tumour was 49%.

In groups 6 and 7, treated s.c. with repeated doses of 5 mg PHIM 106/kg and 12.5 mg PHIM 106/kg, the reduction of the tumour was 53% and 69%.

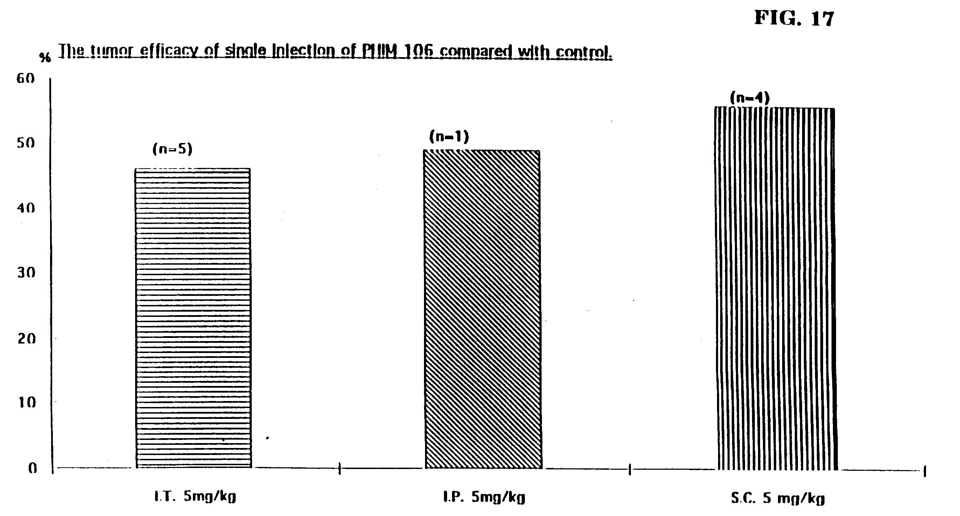

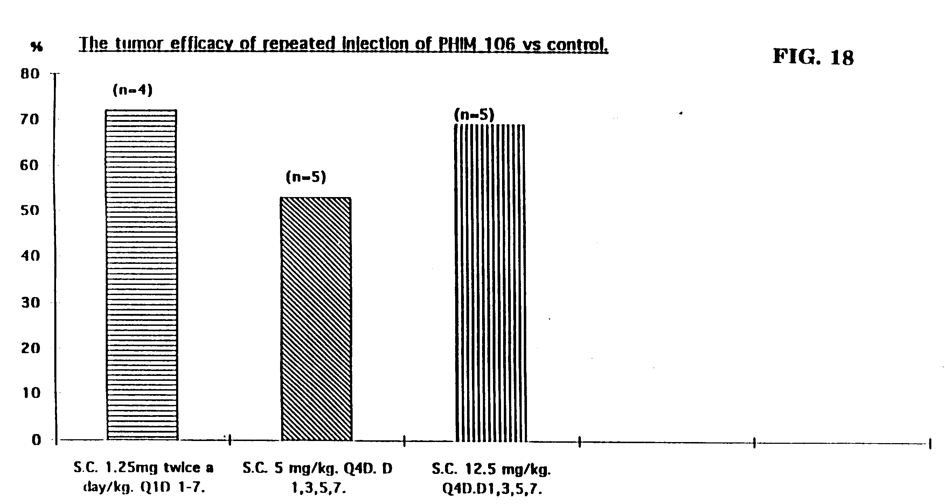

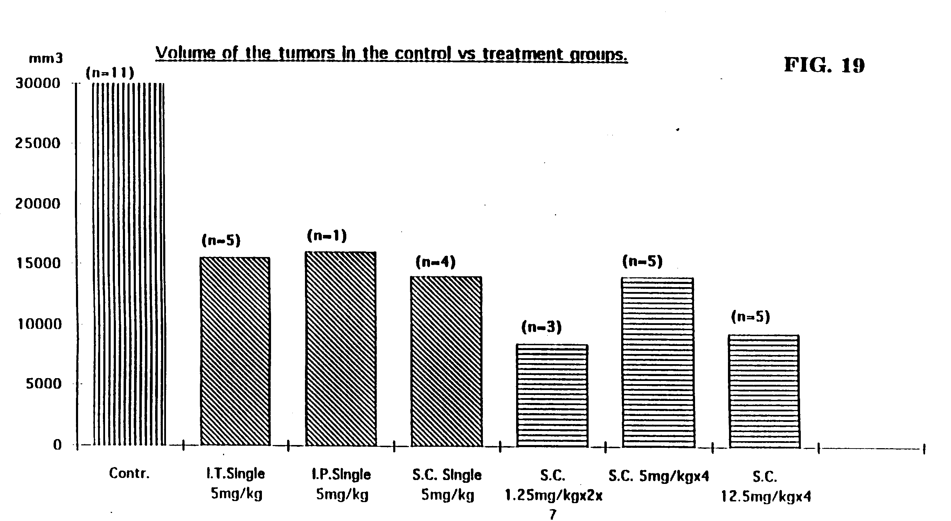

The best efficacy was seen in group 5 treated with repeated doses during 7 days. The reduction of the size compared with the control was 72%. See Figs. 17, 18 and 19.

In all the groups which received treatment more than 50% of the size of the tumours is very necrotic compared to the tumours in the control group. See Pict. 1 and 2.

Within 24 hours after the injection of PHIM 106 the tumours seems to be necrotic.

No adverse reaction could be recognized during or after the treatment.

Within 24 hours from the injection of PHIM, the tumours seem to be necrotic. The size of the necrotic part of the tumours increased and the tumours are smaller and more distinct compared to the control rats. See Pict. 3 and 4.

Differences of body weight could been detected between the control group vs the rats which received treatments. The rats in the control group increased more than the rats in the treatment groups.

No adverse reaction could be observed during or after the treatment.

A suitable dose range for this indication is 0.1 to 500, preferably 1 to 300 mg per treatment.

### Discussion

The number of rats was limited and therefore no statistical calculation could be performed. However, the objective with the study was to investigate the tumor efficacy as such related to the number of doses, the amount of PHIM 106 calculated per kg body weight and to get an indication if PHIM 106 is attacking the tumour when used in different routes of administration.

Overall impression of usefulness was naturally also a very important objective for the study.

Cancer cells are divergent from the immunological points ofview and PHIM 106 should therefore, according to the above, hypothesis about the recognizing and targeting properties of divergent cells, attack and destroy the tumour cells, irrespective of the mode of administration. The results indicate that the tumour efficacy is strong and that the degree of efficacy is similar when it is injected i.p., i.t. or s.c.

Repeated doses of PHIM 106 are better than a single dose, as could be expected because the Yoshida Sarcoma tumour is very fast growing.

The reduction of the tumours is greater in the groups which received 12.5 mg PHIM 106/kg than in the groups which got 5 mg PHIM 106/kg.

On the other hand, the rats which were treated every day for 7 days with a total amount of 17.5 mg PHIM 106/kg had little better efficacy than the rats which were treated every second day with a total of 4 injections and a total of 50 mg PHIM 106/kg.

Yoshida Sarcoma is very malignant and fast growing. Therefore, it could be expected that treatment every day should give better results. All the rats in the treatment groups are in good condition and they had together an increase in body weight of about 10 gr compared with rats in the control group which increased the body weight with about 24 gr. The only explanation for that should be the very large tumour burden. The tumours in the treatment groups are very distinct and solid compared to the tumors in the control group which are spread out into the stomach etc. This also means that there have been problems in measuring the whole size of the tumour in the control group, because the tumours cover a large surface. It is interesting to notice, that one rat in the group treated with 12.5 mg PHIM 106/kg had no tumour growth at al. The tumour had decreased by about 15% compared with the size at the staging day.

Histopathological examination of the tumour in the control group and in the test groups clearly shows that the treated rats with PHIM 106 were very necrotic compared of the untreated rats.

The fact that PHIM 106 was active when administered in three different routes, shows that PHIM 106 is targeting the tumour both from the vein and artery. The subcutaneous administration is of special interest, because PHIM 106 must have been taken up by the lymphatic vessels and will partly reach the tumour via the lymphatic route. The lymphatic uptake of PHIM 106 is also very important for destroying metastasis which partly are spread out that way. The evidence for decreased metastasis could also been seen in the treatment groups compared with the control group. In the control group lungs, intestine, liver large amount of metastasis could be recognized. In the treatment groups over 95% of the rats were free from detectable metastasis. The remaining 5% of the rats had only a few small metastasis in the intestine area.

Even if the number of animal was rather small in the different groups, it should be emphasized that every rat responded to the treatment. The possibility of using a drug for treatment of cancer without any restriction of the methods of administration will, in combination with lack of observable side effects, place PHIM 106 in a new category of anticancer drugs, presumably depending on the natural targeting properties thereof.

The antimicrobial effects of PHIM 106 observed in open pilot studies, will also be of additional importance for the treatment of cancer patients suffering from opportunistic infections. Yoshida Sarcoma is much more malignant than the known tumours in man.

### Example 50 - Navel treatment

A 4 days old boy was treated with PHIM 106 in order to remove necrotic tissue and to avoid upcoming infection.

For the treatment a gauze was used, which had been saturated with a solution of PHIM 106, about 2 mg/piece of gauze. The gauze was wound around the very navel-string so that it covered both healthy navel-string and the somewhat infected as well as the necrotic part of the navel-string. The bandage was changed 4 times per 12 hours. After about 12 hours PHIM 106 has removed the necrotic and the injected part, whereas the healthy part of the navel-string was completely uneffected. No degradation of the healthy skin on the navel-string could be observed, neither could any infection or any skin irritation or inflammation be observed. The healthy navel was treated 4 more times.

Day 6 after the birth the baby was checked at the hospital and it was found that the baby had a very fine navel without any infection, much to the surprise of the doctor and in contrast to the majority of the babies who were examined on the same occasion.

A suitable dose range for this indication is 0.01 to 100, preferably 1 to 25 mg per treatment.

### Example 51 - Infected wound

A 3 months old boy who had surgery for hydro cele och scrotal hernia showed 10 days after surgery a serious infection in the surgery wound in the form of puss formation. Parts of the surgery wound was about to crack after the stitches have been removed.

The boy was treated for 3 days with dressings which had been soaked in PHIM 106,4 mg per treatment. After 3 days of treatment the entire infection was gone and the wound had healed.

The entire operation wound had a length about 15 cm.

A suitable dose range for this indication is 0.01 to 100, preferably 1 to 25 mg per treatment.

### Toxicity on mice

It has not been possible to demonstrate any toxicity of PHIM on humans or animals, despite administration of extreme overdoses, up to about 100 times the corresponding effective dose. The toxicity of PHIM has also been tested on mice with s.c. implanted P388 murine leukemia (a chemically induced cancer) and compared with doxorubicin, a well known anti-cancer drug, abbreviation DOX. The results are summarized in the following Table. It can be seen that no mouse in the PHIM group or the control group died or lost weight, whereas all mice in the doxorubicin group, except for the lowest dosage, lost weight and died. It may be worth mentioning that the highest dose of PHIM (20 mg/kg) is far higher than any of the curing doses used in any of the clinical examples which have been reported earlier in this description.

| **Toxicity of PHIM and DOX administered i.p. daily during 9 consecutive days to mice with P388 murine leukemia** | | | |
|---|---|---|---|
| Drug | Dose (mg/kg) | Toxic death | Body weight change (g) |
| Control | 0 | 0/6 | + 2.3 |
| | | | |
| PHIM | 20 | 0/6 | + 3.3 |
| | 10 | 0/6 | + 3.3 |
| | 5 | 0/6 | + 3.3 |
| | | | |
| DOX | 5 | 6/6 | - 2.3 |
| | 2.5 | 6/6 | - 0.6 |
| | 1.25 | 0/6 | + 0.5 |

### Dosage - general

As noted in connection with the above reported toxicity tests, no toxic effects have been observed despite very heavy overdosage. This is not only true in the case with murine leukemia, but also in all of the clinical examples. Not either has any reduction of the therapeutical effect due to overdosage been observed in any of the clinical examples. In other words, the upper dosage limit is so much higher than the effective dose, so virtually any (reasonable) dosage can safely be used.

The smallest effective dose varies somewhat depending on the condition to be treated, and the presently preferred dosages for the various indications are as follows.

### Administration routes and formulations

The substances of the invention can be safely used in human and animal therapy by virtue of their negligible toxicity.

The therapeutic regimen for the different clinical indications must be adapted to the type of pathology taking into account, as usual, also the route of administration, the form in which the compound is administered and the age, weight and conditions of the subject involved.

The substances of the invention can be applied or administered in the form of solutions. The solutions can be administered e.g. topically (superficial wounds, intact skin); by instillation (urinary tract, fistules); in the form of eye-drops; as a rinsing solution; by inhalation; by injection (intraarticularly, intraarterially, intravenously, intraperitoneally, subcutaneously, intramuscularly); and nasally.

The substances of the invention can also be administered in the form of gels, e.g. topically and by instillation (fistules, decubitus wounds).

When in dry powder form, the substances can be administered e.g. topically, intestinally in acid-resistent capsules and by inhalation.

Dry powder of the substances of the invention is contained in ampoules, each containing either 15 cu of Single-Protein or 25 cu of Multi-Protein. In order to prepare a ready-to-use solution the contents of one ampoule are reconstituted with sterile sodium chloride solution or sterile water to form solutions containing from 0.10 to 25 cu of Multi-Protein/Single-Protein.

When using the substances of the invention in the form of a gel, powdered Multi-Protein or Single-Protein ex temporere is formulated with a hydrogel to the desired concentration to form a ready-to-use hydrogel. The hydrogel as such consists of low molecular weight hydrolyzed starch containing >90% of water.

From our studies it can be concluded that, by using the above administration routes, the substances of the invention find their way through the blood system, arterielly and venously, and through the lymphatic system. In the topical administration route there is a direct contact between the enzymes and its substrate.

### PHARMACUETICAL FORMULATIONS

### Powder-Ampoules

15 cu of Single-Protein or 25 cu of Multi-Protein are filled into a conventional ampoule using known technique.

### Ready-to-use solution

The contents of an ampoule (cf. above) are reconstituted with sterile sodium chloride solution (physiological saline) or sterile water to a concentration of Multi- or Single-Protein of from 0.10 to 25 cu/ml.

### Hydrogel

A hydrogel consisting oflow molecular weight hydrolyzed starch containing >90% of water is prepared in a way known per se. Upon preparation of the gel it is packed in portions and the packages are sterilized (autoclaving).

### Ready-to-use Hydrogel

Powdered Multi-Protein/Single-Protein is formulated ex temporere with the Hydrogel (cf. above) to the desired concentration, e.g. 2.5 or 5 cu/ml.

## Claims

1. Use of an enzyme composition comprising essentially of a single proteolytic enzyme having a molecular weight from 26,000 to 32,000, as determined by SDS-PAGE and isolated from antarctic krill, wherein the composition exhibits both endo- and exo-peptidase activity, for the manufacture of a composition for removing dental plaque.

2. A method of removing dental plaque comprising contacting the dental plaque with a dental plaque removing effective amount of an enzyme composition comprising essentially of a single enzyme having a molecular weight from 26,000 to 32,000, as determined by SDS-PAGE and isolated from antarctic krill, wherein the composition exhibits both endo- and exo-peptidase activity.
